Europäisches Patentamt

European Patent Office

Office européen des brevets

(19)

(11) Publication number: **0 119 702**
**A2**

(12) # EUROPEAN PATENT APPLICATION

(21) Application number: **84300658.6**

(22) Date of filing: **02.02.84**

(51) Int. Cl.³: **G 01 N 33/54**

(30) Priority: **14.02.83 US 466329**

(43) Date of publication of application: **26.09.84**
**Bulletin 84/39**

(84) Designated Contracting States: **AT BE CH DE FR GB IT LI LU NL SE**

(71) Applicant: **Sen, Arup, 10992 Ashton Avenue, 204, Los Angeles California 90024 (US)**
Applicant: **LERNER, Richard A., 7750 East Roseland, La Jolla CA 92037 (US)**
Applicant: **Houghten, Richard A., 558 Ford Avenue, Solana California 92075 (US)**
Applicant: **BITTLE, James L., 5353 Calle Vista, San Diego CA 92109 (US)**

(72) Inventor: **Sen, Arup, 10992 Ashton Avenue, 204, Los Angeles California 90024 (US)**
Inventor: **LERNER, Richard A., 7750 East Roseland, La Jolla CA 92037 (US)**
Inventor: **Houghten, Richard A., 558 Ford Avenue, Solana California 92075 (US)**
Inventor: **BITTLE, James L., 5353 Calle Vista, San Diego CA 92109 (US)**

(74) Representative: **Grundy, Derek George Ritchie et al, CARPMAELS & RANSFORD 43, Bloomsbury Square, London WC1A 2RA (GB)**

(54) **Synthetic polypeptides from viral oncogenes.**

(57) Synthetic polypeptides and their conjugates bound to a carrier are disclosed along with antibodies raised thereto, and methods of their use. The amino acid residue sequence of the synthetic polypeptides correspond substantially to an amino acid sequence of a determinant of an oncoprotein encoded by an oncogenic virus. That determinant domain is vicinal to but exclusive of an active site of the oncoprotein. The synthetic polypeptide or its conjugate is capable of inducing the production of antibodies which can inhibit a reaction at an oncoprotein active site, immunoprecipitate the oncoprotein and react competitively with an admixture containing the synthetic polypeptide or its conjugate and another oncoprotein having a homologous determinant domain.

-1-

SYNTHETIC POLYPEPTIDES FROM VIRAL ONCOGENES

DESCRIPTION

The present invention relates to chemically synthesized polypeptides and their conjugates bound to a carrier, and more specifically to antigenic, synthetic polypeptides and their conjugates that substantially correspond to a determinant portion of a protein encoded by both of two oncogenes.

Background

Oncornaviruses are a family of viruses which can cause various types of neoplasia (e.g., leukemia, lymphomas, sarcomas and alike) in birds and lower animals such as mice and cats. The largest number of oncornaviruses are retroviruses which contain a single strand of RNA as the genomic material rather than DNA.

The single-stranded RNA genome of each of these viruses gives rise to a double-stranded DNA molecule after the virus infects a susceptible host. This DNA replica of the viral genome then introduces itself permanently into a chromosome of the successfully infected cell and replicates in that host chromosome.

Some members of the retrovirus family are highly oncogenic as judged by their ability to cause the formation of solid tumors within a short period of time after being inoculated into the host. These viruses can also cause "cancerous" changes in cells grown in culture in the laboratory; such changes are called "transformation" and provide a reliable in vitro biological assay for oncogenic viruses. Several such viruses have been isolated from chickens, turkeys, mice, rats, cats and monkeys.

A single gene, the oncogene, located on the genome of these highly oncogenic viruses is responsible for the tumorgenic potential of the virus. In the case of several viruses, the protein products of their oncogenes, referred to herein as oncoproteins, have been immunologically identified by taking advantage of the fact that serum from an animal bearing a virus-induced tumor contains antibodies directed against the oncoprotein that is encoded by the oncogene of the virus.

A rapidly growing body of evidence indicates that the oncogenes of retroviruses are closely related to and derived from specific genetic loci in the normal cellular genetic information of all vertebrates. Molecular hybridization studies using specific nucleic acid probes during the middle 1970's, followed by genetic cloning of viral oncogenes and their cellular relatives by recombinant DNA technology, have established the kinship between retroviral oncogenes (v-onc) and cellular oncogenes (c-onc) found in all normal vertebrate cells.

The cellular oncogenes are present in many species that are evolutionarily related while the viral oncogenes are usually specific as isolated from particular species. Moreover, the homology between the v-onc and the cellular DNA is believed to be the highest for species of origin of a particular virus. These observations suggest that retroviral oncogenes, represent escaped and/or partially metamorphosed cellular genes that are incorporated into the genomes of transmissible, infectious agents, the retroviruses.

Molecular analysis of the nearly two dozen retroviruses thus far isolated has revealed more than a dozen different oncogenes, each distinguished by its nucleotide sequence, and each with a corresponding cellular oncogenic homolog. Although the number of such viral oncogenes appears to be growing nearly in parallel with the isolation of new oncogenic retroviruses, the ultimate number may not be very large. For example, the same v-onc for a c-onc designated fps of avian origin is represented at least twice among a limited number of avian retrovirus isolates; its mammalian cognate designated fes in feline species is found in two different strains of feline sarcoma viruses.

Regardless of the total number ultimately found, c-onc genes might constitute a multigene family as is the case for genes of many polypeptide hormones, the immunoglobulins, histocompatibility antigens and the like. The apparent structural individuality of members of the oncogene family, as described in terms of nucleotide sequences might obscure common origins and related functions.

Oncoproteins encoded by at least eight different members of a family of only about two dozen different viral oncogenes, exhibit a common function. Namely, they have the ability to catalyze a phosphotransfer reaction, the phosphorylation of selective proteins, particularly at the amino acid tyrosine. These phosphorylation reactions include autophosphorylation.

Oncoproteins from other oncoviruses have been shown to take part in additional reaction types. For example, the product of a ras gene has been shown to bind a nucleoside triphosphate, GTP. The product of the oncogene myc has been found to bind DNA in the cell nucleus. [Donner et al., Nature, 296, 262-266(1982); Abrams et al. Cell, 29, 427-439 (1982).]

The fact that normal cells contain a family of oncogenes (c-onc) in their chromosomes clearly supports a mechanism other than infection with oncogenic viruses as the causation of neoplasia in animals and man. This mechanism could include the "activation" of one or more cellular oncogenes by environmental carcinogens and by other mutational events. Experimental evidence clearly supports the concept that the activation of already characterized and as yet unidentified cellular oncogenes can be associated with a neoplastic state in mammalian and avian cells.

In examining the retroviruses more closely, one notes that they are comprised primarily of a single strand RNA surrounded by a protein coat. Upon infection of a host cell, the viral RNA is transcribed "backwards" into DNA by an enzyme called reverse transcriptase. The thus produced DNA is thereafter integrated into the infected cell's genome with the result that the viral genes are replicated along with cellular genes, and the protein products coded for by the viral gene are produced by the host cell. It is noted that the protein coded for by the viral oncogene and having a corresponding, homologous gene within the host cell is generally not present in the virus particle that infects the cell, but is only expressed after infection.

The transformation of cells in culture provides evidence that a virus contains an oncogene, and that the oncovirus can induce neoplasia in vivo in some cells of an animal host. The ability of a virus to transform a cultured cell is used herein as a defining characteristic of an oncogenic virus. Viruses which do not transform cells in culture have been found to lack oncogenes and induce tumors by a different mechanism.

Thus, a picture emerges in which an oncogenic virus bearing an unexpressed gene for a protein infects the cell and causes the cell to produce, inter alia, the oncoprotein which was unexpressed in the virus. The infected cell also contains a normally present gene for an oncoprotein. At some time in its existence, the cell can produce an oncoprotein which is homologous to the oncoprotein encoded by the infecting oncovirus. Recent work has illustrated that the protein encoded by the cellular oncogene, c-onc, can be found in small quantities in healthy cells, and that cellular protein is also termed an oncoprotein.

One current theory holds that the difference between a normal cell and an oncogenically transformed or neoplastic cell resides in the effect caused by the increased amount of the naturally occurring oncoprotein that is encoded by both the viral oncogene and the cellular oncogene. Thus, if the production of the oncoprotein could be reduced or if its effect upon the cell, e.g., phosphorylation could be reduced toward the normal level, the neoplasia of the infected cell could be reduced or eliminated.

Discussions concerning the present state of the art relating to oncogenic retroviruses can be found in the following articles: Bishop, Cell, 23, 5-6 (1981); Bishop, Scientific American, March, 81-92 (1982); Cooper, Science, 218, 801-806 (1982); and Weiss, Nature, 299, 9-10 (1982).

Viral and cellular oncogenes are of interest scientifically, but are of little value in themselves in providing treatments and diagnostic reagents for neoplastic conditions. The oncprotein encoded by a cellular or viral oncogene is also of interest, but is difficult to prepare in useful quantities, and that preparation, even when carried out using the sophisticated tools of genetic engineering, can lead to proteins that are contaminated with products from the cellular manufacture of that protein.

On the other hand, it has been recently found that a pathogen-related protein can be immunologically mimicked by the production of a synthetic polypeptide whose sequence corresponds to that of a determinant domain of the pathogen-related protein. Such findings are reported by Sutcliffe et al., Nature, 287, 801-805 (1980); Doolittle et al., Proc. Natl. Acad. Sci. U.S.A, 77, 5197-5201 (1980); Lerner et al., Proc. Natl. Acad. Sci. U.S.A., 78, 340-347 (1981) and Baltimore et al., Cell, 28, 395-404 (1982). Such synthetic antigens are also disclosed in the co-assigned patent application Serial No. 248,059 to Lerner et al., filed March 27, 1981. (European Patent Specification No. 0044710).

The present invention is directed to certain synthetic polypeptides that have special characteristics and properties, and to products utilizing those synthetic polypeptides. While the present synthetic polypeptides and the novel products made therefrom can be prepared using the general technology disclosed in the above documents, the synthetic polypeptides themselves, their unique properties, products made from and with them and their methods of use are neither taught nor suggested in the above documents.

Brief Summary of the Invention

A synthetic polypeptide in accordance with this invention has an amino acid residue sequence corresponding substantially to an amino acid sequence of a determinant domain of an oncoprotein encoded by an oncogenic virus. The determinant domain, including the amino acid sequence selected to be synthesized, is vicinal to but exclusive of an active site of the oncoprotein. This polypeptide in its entirety is free from an amino acid residue sequence that corresponds to that oncoprotein active site, but either alone, or as a conjugate bound to a carrier has the capacity to induce the production of antibodies when injected into a host.

The antibodies so produced, which are also encompassed by this invention as are smaller portions thereof containing idiotypic regions, inhibit a reaction at that oncoprotein active site, such as the catalysis of phosphotransfer. These antibodies are also capable of immunoprecipitating the oncoprotein when admixed with it. In addition, these antibodies can react competititvely with an admixture containing the synthetic polypeptide or its conjugate and another oncoprotein having a homologous determinant domain present in a body component of a tumor-bearing animal; the tumor being other than that induced by the oncogenic virus.

The above polypeptide can also be used as a portion of a diagnostic composition for detecting the presence of neoplastic diseases, as well as in a method for inhibiting the growth of neoplastic cells and in vaccinating cells against neoplastic growth.

A diagnostic reagent system embodying this invention is useful for the determination of the presence of an increased amount of oncoprotein compared to the amount of that oncoprotein normally present. This system comprises in separate containers (a) a first reagent and (b) a second, reagent, both in biologically active form, along with indicating groups.

The first reagent contains the above described synthetic polypeptide, or a conjugate prepared therefrom. The second reagent includes polyamides containing idiotypic regions of antibodies raised to the synthetic polypeptide or its conjugate. Indicating groups are also provided to the system and can be initially bound to or free from either of the two reagents.

Admixture of predetermined amounts of the first and second reagents in the presence of a predetermined amount of a body component to be assayed results in an amount of immunoreaction. The amount of the immunoreaction so produced is different from a known immunoreaction amount when an increased amount of oncoprotein is present in the body component. The amount of immunoreaction is typically diminished when an increased amount of the oncoprotein is present in the body component, compared to the usual amount present. Exemplary diagnostic reagent systems include enzyme-linked immunosorbent assays (ELISA) wherein the indicator group is an enzyme such as horseradish peroxidase which is bound to the above antibody or another antibody, and radioimmunoassays in which the indicating group is a radioactive element such as $^{125}I$ present in either the synthetic polypeptide or the antibody raised thereto.

A particular benefit of the present invention is that its products and methods can be utilized to inhibit the growth of neoplastic cells. Thus, the growth of neoplastic cells having cellular or viral oncoproteins on their surfaces can be inhibited by contacting the neoplastic cells with an effective amount of the above-described antibodies, and maintaining that contact to form immune complexes between the antibodies and oncoprotein molecules.

Another of the benefits of the present invention is that its products and methods can be used to vaccinate animals against neoplastic cell growth. Here, a vaccine containing the synthetic polypeptide described above or a conjugate prepared from the polypeptide is provided in a physiologically tolerable vehicle. The vaccine is then injected in an effective amount into the bloodstream of the animal to be immunized.

The present invention provides several additional advantages and benefits. One advantage of the present invention is that use of the synthetic polypeptide obviates the need for the presence of its corresponding intact oncoprotein. Consequently, impurities such as cellular debris and toxins that are associated with the production of usable amounts of viral proteins from bacteria are absent from the product of this invention.

Another benefit of the present invention is that its products and methods can be used to detect the presence of neoplastic cells in a host.

Still further advantages and benefits of the present invention will become apparent to those skilled in the art from the detailed description, examples and claims which follow.

Brief Description of the Drawings

In the drawings, constituting a portion of this disclosure

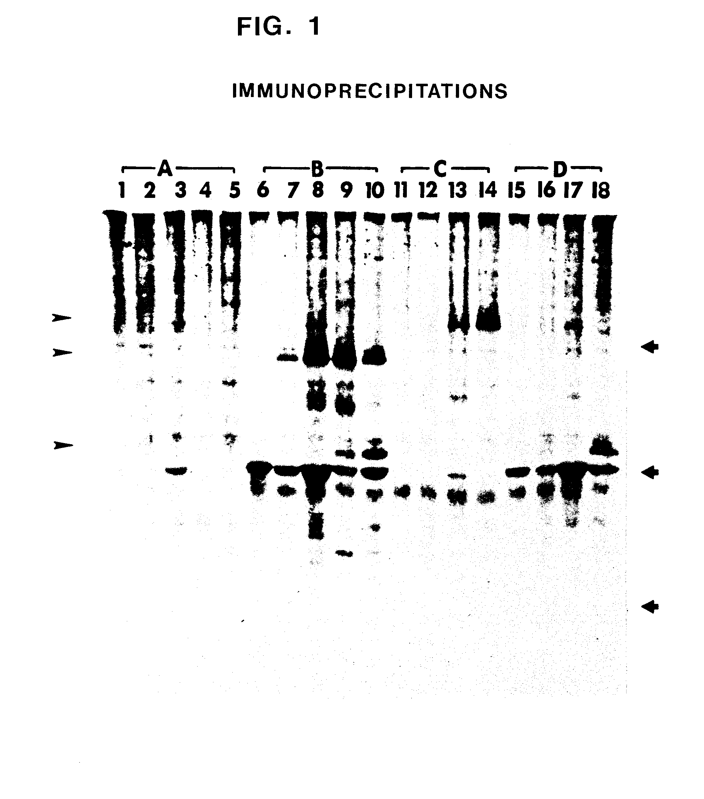

Figure 1 is a photograph of an autoradiogram illustrating a polyacrylamide gel electrophoresis in the presence of sodium dodacyl sulfate (SDS-PAGE) analysis of immunoprecipitates of metabolically labeled extracts of FeSV transformed cells. Uninfected normal cells and FeLV (type B) infected or FeSV transformed mink cells were labeled for 2 hours with $^{35}$S-methionine and cell extracts were precleared with preimmune rabbit serum. One hundred microliters of precleared lysate from each cell line

0119702

was incubated with either preimmune serum (lanes 1, 6, 11 and 15), antiserum 5014 directed against the 30-mer synthetic polypeptide I (lanes 2, 7, 12 and 16), antiserum 5016 directed against the 12-mer synthetic polypeptide II (lanes 3, 8, 13 and 17), antiserum against FeLVpl5 (lanes 4 and 9), or an antiserum directed against FeLVp27 (lanes 5, 10, 14 and 18). Antibody-antigen complexes formed during the incubation were precipitated using Staphylococcus A bacteria. The precipitated complexes so formed were washed, and were then dissociated into their component parts. The proteins were thereafter analyzed under reducing-denaturing electrophoresis through a polyacrylamide gel matrix.

The labeled extracts were prepared from: panel A) Normal mink CCL64 line, panel B) ST-FeSV transformed FeLV type B producer mink line, panel C) GA-FeSV transformed nonproducer mink line, and panel D) FeLV type B infected nontransformed mink line. The immune complexes were electrophoresed on a 20 centimeter long 10% Laemmli gel. [Laemmli, Nature, 227, 680-685 (1970).]

The migration of standard molecular weight markers indicated by arrows on the right-hand side are (from top to bottom): phosphorylase A (95 Kd), rabbit immunoglobulin G heavy chain (55 Kd), and ovalbumin (44 Kd). Only the top 12 centimeters of the gel is shown; there were no significant labeled protein bands below this portion of the gel. The pointers on the left indicate the position of the 108 Kd GA-FeSV polyprotein (upper pointer; lanes 13 and 14), the 85 Kd ST-FeSV polyprotein (middle pointer; lanes 7, 8, 9 and 10) and a 65 Kd FeLV gag-precursor (lower pointer; lanes 9, 10 and 18).

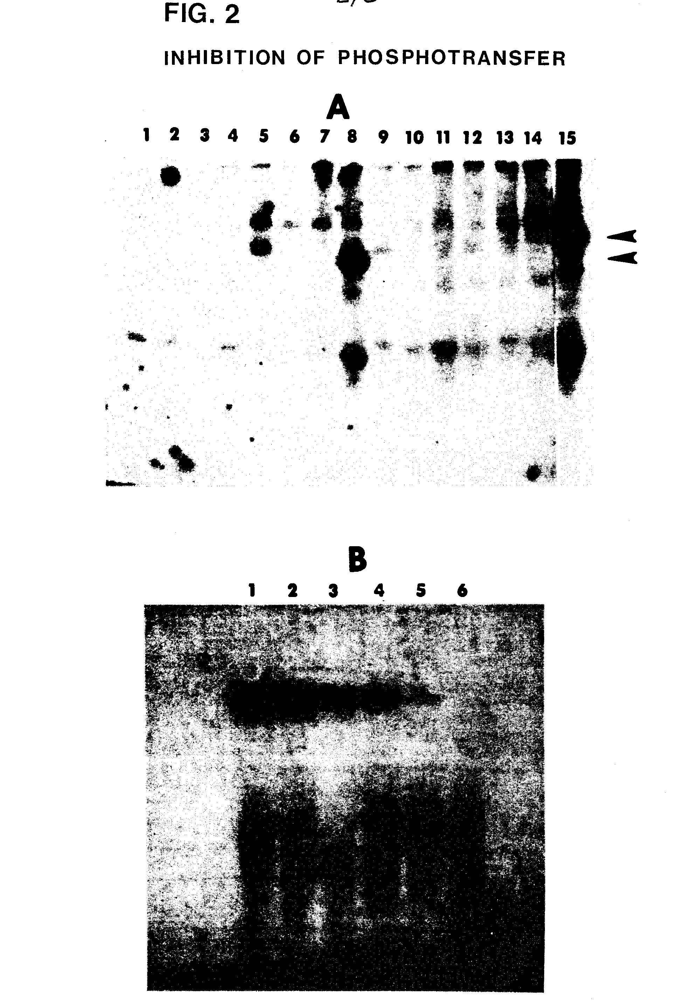

Figure 2A is a photograph of an autoradiogram illustrating SDS-PAGE analysis of products phosphorylated by incubation of immune complexes with gamma-$^{32}$P-labeled ATP. Immune complexes were prepared from unlabeled lysates of normal mink cells (lanes 1 through 4), ST-FeSV transformed FeLV producer mink cells (lanes 5 through 8), FeLV-infected untransformed mink cells (lanes 9 through 11) and GA-FeSV transformed nonproducer mink cells (lanes 12 through 15). Immune complexes bound to formalinized Staphyloccus A were incubated with 2 microCuries of gamma-$^{32}$P-ATP (approximately 3,000 Curies/micromole) in 10 millimolar Tris-HCl (pH 7.4)/5 millimolar MgCl$_2$ for 20 minutes at 22°C. The phosphorylated proteins were dissociated by boiling in SDS-PAGE sample buffer and electrophoresed on a 10 centimeter long 10% acrylamide gel. The sera used in the immunoprecipitation were as follows: Preimmune rabbit serum, lanes 1, 5, 9 and 12; Antiserum 5014 against the 30-mer synthetic polypeptide I, lanes 2, 6 and 13; Antiserum 5016 against the 12-mer synthetic polypeptide II, lanes 3, 7, 10 and 14; Antiserum against FeLVp27, lanes 4, 8, 11 and 15. (The immune complex kinase activity from the GA-FeSV nonproducer was much higher than the incorporation in the adjacent lanes; as such, lane 15 of this gel was exposed separately and the autoradiogram was aligned after developing the x-ray film.) The pointers on the right hand side indicate the positions of the GA-FeSV p108 (top; lane 15) and the ST-FeSV p85 (bottom; lane 8).

Figure 2B is a photograph of an autoradiogram illustrating SDS-PAGE analysis of the products of in vitro phosphorylation in immune complexes prepared using varying amounts of anti-FeLVp27 and antipolypeptide II serum. Lysates from ST-FeSV transformed cells were precleared with preimmune rabbit serum. Fifty microliter aliquots (equivalent to approximately $2 \times 10^4$ cells) were incubated with the following serum mixtures:

1. 5 microliters 1:25 diluted anti-FeLVp27 + 15 microliters preimmune serum;

2. 5 microliters 1:25 diulted anti-FeLVp27 + 12.5 microliters preimmune serum + 2.5 microliters antiserum 5016;

3. 5 microlites 1:25 diluted anti-FeLVp27 + 10.0 microliters preimmune serum + 5.0 microliters antiserum 5016;

4. 5 microliters 1:25 diluted anti-FeLVp27 + 7.5 microliters preimmune serum + 7.5 microliters 5016;

5. 5 microliters 1:25 diluted anti-FeLVp27 + 5.0 microliters preimmune serum + 10.0 microliters 5016; and

6. 10.0 microliters preimmune serum + 10.0 microliters 5016.

Immune complexes were washed and incubated with $^{32}$P-ATP as before. The $^{32}$P-labeled products formed after a 20 minute incubation at 22°C were analyzed on a 10% discontinuous Laemmli gel.

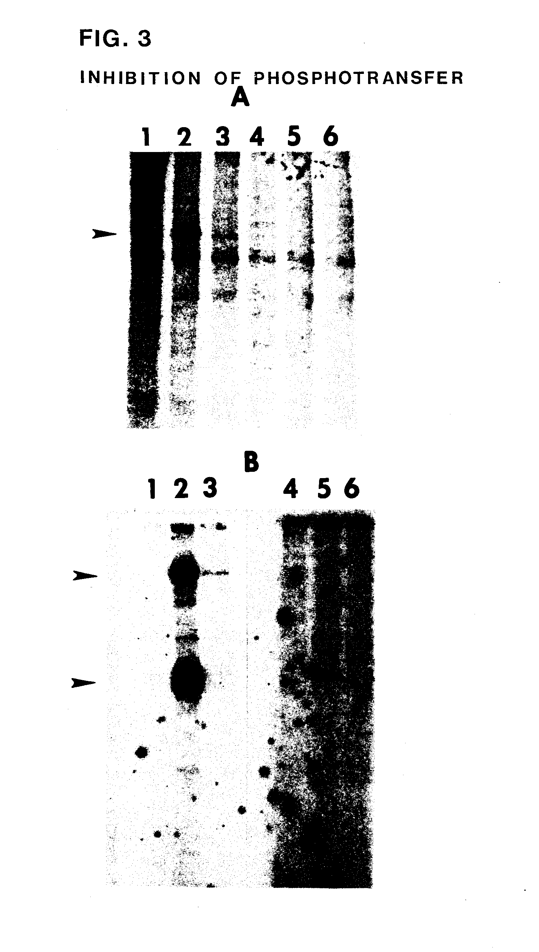

Figure 3A is a photograph of an autoradiogram illustrating SDS-PAGE analysis of immunoprecipitates from metabolically labeled Fujinami sarcoma virus (FSV) transformed rat kidney (NRK) cells and untransformed NRK cells. $^{35}$S-methionine labeled cell extracts from FSV transformed NRK cells (lanes 1 through 3) and the normal parent NRK line (lanes 4 through 6) were immunoprecipitated with preimmune rabbit serum (lanes 1 and 4); antiserum 5016 against v-fes synthetic polypeptide II (lanes 2 and 5); or antiserum 5014 against v-fes synthetic polypeptide I (lanes 3 and 6). The immunoprecipitates were analyzed on a 10 centimeter long 10% Laemmli gel. The Figure shows only the top one-half of the gel. The pointer on the left indicates the expected position of fps p130 oncoprotein.

Figure 3B is a photograph of an autoradiogram illustrating SDS-PAGE analysis of products phosphorylated in vitro in immune complexes made with anti-v-fes synthetic polypeptide sera and extracts from NRK or FSV transformed NRK cells. Immune complexes were made with unlabeled extracts from FSV transformed cells (lanes 1 through 3) or normal NRK parent cells (lanes 4 through 6) and incubated with $^{32}$P-ATP as discussed above. The sera used in these studies were: Preimmune rabbit serum - lanes 1 and 4; serum 5016 against v-fes synthetic polypeptide II - lanes 3 and 6; an RSV tumor-bearing rabbit serum (a gift from Dr. Joan Brugge, State University of New York, New York) which cross-reacts with the Fujinami-fps oncoprotein - lanes 2 and 5. The pointers on the left indicate the positions of the v-fps p130 (top) and immunoglobulin H-chain (bottom). The portion of the gel containing samples from normal NRK line was exposed five times as long as the other half containing transformed cell samples. Upon even longer exposure, a faint band of radioactivity in the immunoglobulin H-chain region could be seen in lane 5 presumably reflecting the kinase activity of the cellular oncoprotein encoded by the cellular src oncogene (c-src).

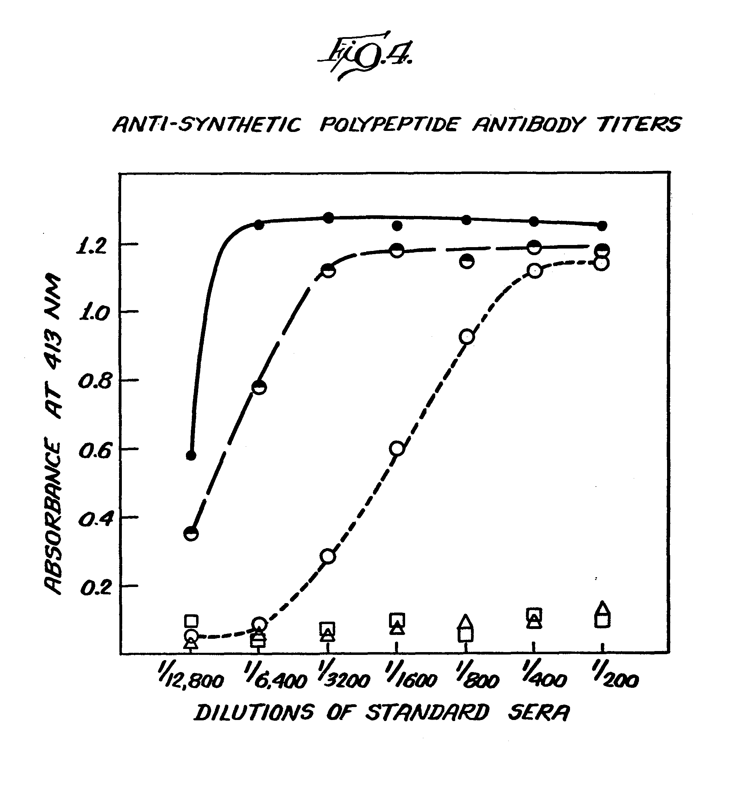

Figure 4 illustrates measurement of anti-polypeptide antibody titer in rabbit sera by an enzyme-linked immunosorbent assay (ELISA).

Approximately 10 picomoles of the 12-mer polypeptide II were absorbed onto solid supports provided by each well of 96-well Falcon microtiter plates. Serial dilutions of different test sera from immunized and control rabbits were allowed to bind to these wells. After washing unbound material, a peroxidase conjugate of goat antisera to rabbit immunoglobulin was added to the wells. After washing unbound material, the amount of peroxidase bound to each well was measured by a colorimetric assay using o-phenylenediamine and hydrogen peroxide. The results were plotted as serum dilution (horizontal axis) versus color intenstiy (vertical axis). The symbols are as follows:

□—□ Preimmune rabit serum (control);

△—△ Serum from a rabbit immunized with a heterologous peptide (control);

○—○ Serum from rabbit five weeks after immunization with 12-mer;

◐—◐ Serum from rabbit seven weeks after immunization with 12-mer; and

●—● Serum from rabbit nine weeks after immunization with 12-mer.

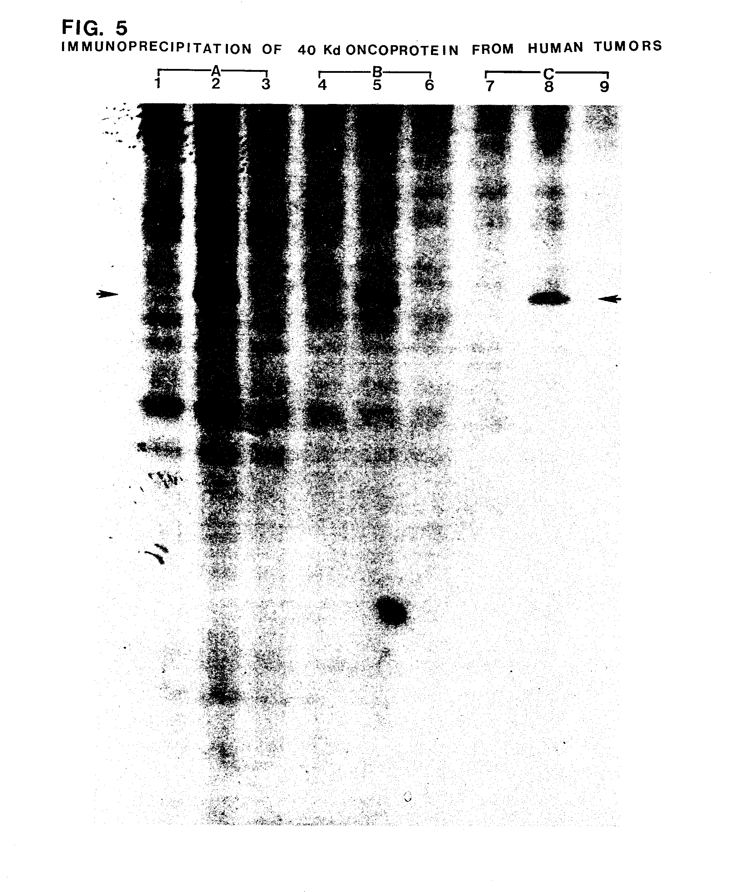

Figure 5 is a photograph of an autoradiogram illustrating the identification of a 40 Kd protein in human tumor cells by immunoprecipitation using antisera to synthetic polypeptide II conjugated to keyhole limpet hemocyanin (KLH). Cultured cell lines obtained from three different human tumors, a carcinoma (panel A), a rhabdomyosarcoma (panel B) and a fibrosarcoma (panel C) were metabolically labeled with the radioactive amino acid, L-methionine ($^{35}$S-labeled). Analysis of immunoprecipitation was as in Figure 1. Lanes 1, 4 and 7 illustrate immunoprecipitates obtained with preimmune rabbit serum. Lanes 2, 5 and 8 illustrate immunoprecipitates obtained with antiserum to the 12-mer polypeptide II. Lanes 3, 6 and 9 illustrate immunoprecipitates obtained with antiserum against an unrelated polypeptide conjugated to KLH. The arrows point to the position of the 40 Kd protein.

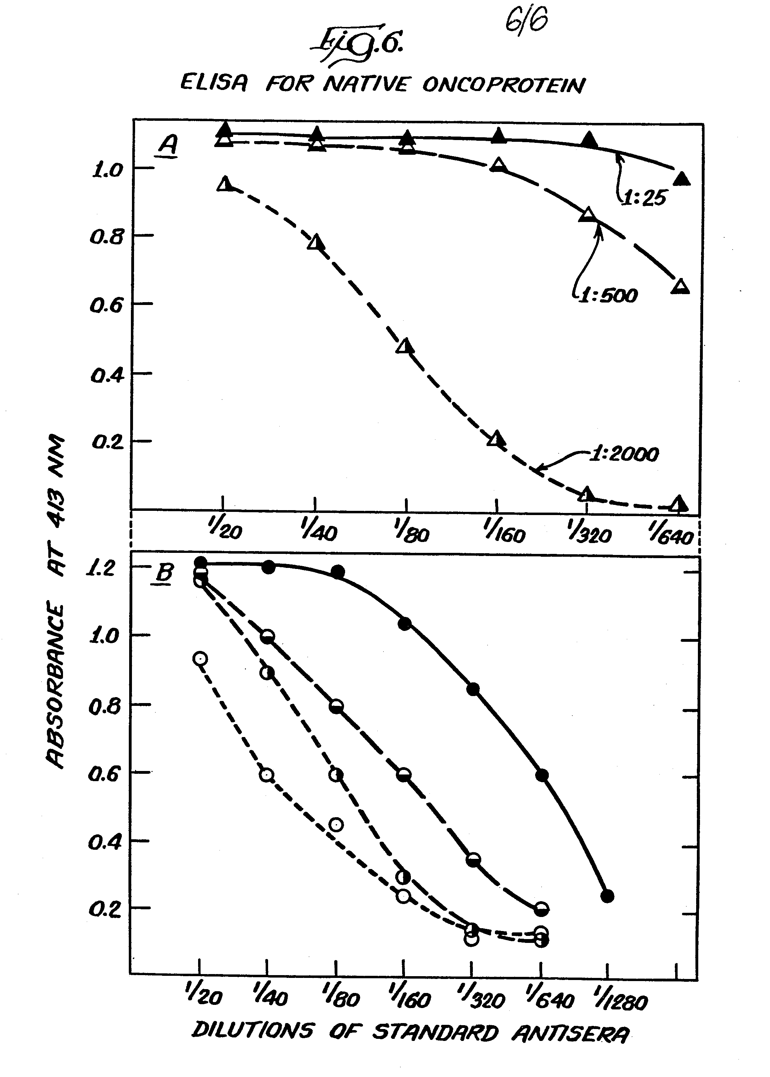

Figure 6 illustrates the development of a competition ELISA for qualitatively detecting and quantitatively measuring the amount of oncoprotein in a sample to be analyzed using synthetic polypeptides of this invention and antisera directed against them, or their conjugates with KLH. The antisera contained horseradish peroxidase bonded to the antibody molecules. Absorbance readings were obtained as described above.

A. ELISA readings with serial dilutions of a 25-fold ( ▲—▲ ), 500-fold ( ▲—▲ ) and 2000-fold ( ▲—▲ ) diluted stock solutions of rabbit antiserum to synthetic polypeptide II using that polypeptide as an immobilized antigen as described for Figure 4.

B. The 500-fold diluted stock solution of anti-polypeptide serum was incubated with none ( ● ) to increasing amounts ( ◐,◑ and ○) of extracts from cells that had been made cancerous with FeSV. The incubated serum samples were then subjected to ELISA analysis in the presence of polypeptide II as the immobilized antigen. The results show a lowering of the apparent anti-polypeptide antibody titer in the serum as a result of incubation with extracts containing the native FeSV oncoprotein. This lowering is due to the binding of the anti-polypeptide antibody molecules to the native antigen, and as such, the levels of available anti-polypeptide antibody molecules for binding to the synthetic polypeptide on the ELISA plates were lowered.

Detailed Description of the Invention

I. Introduction

Oncogenic viruses comprise a class of viruses, many of which are retrovirsuses having a single strand of RNA as their genomic material. Oncogenic viruses are classified as such in that their genome codes for an oncoprotein which does not represent a major structural component of the virus particle and is expressed in an infected, host cell. The gene which codes for the oncoprotein is defined as the oncogene. Proteins related to the viral oncoproteins are encoded by genes present the host cells' native genomes prior to infection. Those cellular oncoproteins are expressed, at least in some cases, to a small extent in the normal growing cell. Thus, expression of the oncogenes in the infected host cells to produce the oncoproteins provides the infected cells with an increased level of the oncoprotein over that which is normally present.

Oncogenic virsuses may be still further characterized by the fact that the oncoprotein contains an active site through which it interacts with body components as by binding to DNA, binding and cleaving another protein, phosphotransfer, and the like. The term "active site" is used herein to include both the domains of binding and action of the oncoprotein.

An oncoprotein active site may include only one amino acid such as tyrosine-419 of the $pp60^{src}$ oncoprotein or several amino acids. When several amino acids comprise an oncoprotein active site, the distance from a determinant domain to the active site is determined from the proximal amino acid of the determinate domain to the approximate middle of the active site.

Several well studied oncoproteins have been found to catalyze a phosphotransfer reaction, typically from ATP, thereby classifying those oncoproteins enzymatically as kinases. In addition, oncoproteins from different oncovirsuses have been found to have homologous antigenic determinant domains. As a consequence of the homology between antigenic determinant domains, antibodies to a first oncoprotein can be used to immunoreact with a second oncoprotein encoded by a gene of a different oncovirus or by a cellular oncogene.

The present invention makes particular use of the homology between antigenic determinants on different oncoproteins as well as the fact that many oncoproteins have an active site in relatively close proximity (vicinal) to the homologous antigenic determinants. This proximity can be either in the primary oncoprotein sequence or through space due to the tertiary, folded structure of the oncoprotein.

Thus, in the present invention homology of determinants of oncoproteins and the proximity of the homologous determinants to the oncoprotein active site is of import as distinguished from a homology of active sites of the oncoproteins. This distinction is noted because antibodies raised to an oncoprotein active site domain can also cause immunoreactions such as immunoprecipitation of non-oncoproteins that contain a homologous active site. Such indiscriminant immunoprecipitation and reaction with non-oncoproteins can lead to disruption of desirable cell functions in addition to disruption of neoplastic cell functions.

An example of indiscriminant immunoprecipitation caused by antibodies to an oncoprotein's active site can be found in the paper by Wong and Goldberg, Proc. Natl. Acad. Sci. USA, 78, 7412-7416 (1981). Those authors produced antibodies to the phosphotransfer active site of the pp60$^{src}$ oncoprotein of Rous sarcoma virus by using a conjugate prepared from keyhole limpet hemocyanin and a decapeptide that included tyrosine-419, the amino acid residue at the active site of the pp60$^{src}$ oncoprotein that is autophosphorylated, as well as a polypeptide sequence on either side of tyrosine-419.

Wong and Goldberg reported that antibodies prepared to the decapeptide conjugate immunoprecipitated the pp60$^{src}$ oncoprotein, but also caused precipitation of numerous non-oncoproteins from both infected and uninfected cells. Such broad cross-reactivity of antibodies with both oncogene-encoded and non-oncogene-encoded proteins is not desirable, as noted above, and is thought to stem from the use of a conjugated polypeptide sequence that includes the oncoprotein active site, or a portion thereof, which site is homologous with the sites of the other precipitated proteins.

Wong and Goldberg also reported that the interaction between the antipeptide antibodies and the oncoprotein was weak. The reported weakness of the interaction was ascribed to the short length of the synthetic polypeptide, or alternatively, to the active site determinant's being not readily accessible in the native oncoprotein. The following discussion points out that selection of a hydrophilic determinant region of an oncoprotein having about 10 to about 40 amino acids can provide a corresponding sequence for a synthetic polypeptide that induces production of antibodies that cause strong, discriminanting immunoreactions with the original oncoprotein, as well as with oncoproteins from other viral or cellular oncogenes.

The earlier published work of Sen et al., Abstract, p. 94, RNA Tumor Virus Meeting, (May 1981) Cold Spring Harbor Laboratories, Cold Spring Harbor, New York, reported on the preparation of antibodies to a similar polypeptide sequence including an on either side of tyrosine-419 of $pp60^{src}$, and use of those antibodies to form an immune complex with the $pp60^{src}$ oncoprotein, among other proteins, including proteins from non-infected cells.

Work published by Papkoff et al., Cell, 27, 109-119 (1981) and 28, 417-426 (1982) indicated a similar difficulty with non-specific antibodies. Those workers conjugated the carboxy-terminal 12 amino acids of an oncoprotein encoded by the M-MuSV transforming gene v-mos to bovine serum albumin and then raised antibodies to that conjugate. The antisera so prepared immunoprecipitated many major bands from both uninfected and MuSV-transformed cells that were not found in precipitates made with preimmune serum.

## II. Overview Of Useful Techniques

### A. Synthetic Polypeptides

Choice of the synthetic polypeptide to be utilized herein begins with the amino acid sequence of an oncoprotein. That sequence may be determined by several techniques well known in the art and include the classical, wet chemical technique of partial hydrolysis and sequencing of the individual products. The more recent techniques of sequencing of the genome from either the virus or an infected host that encodes the oncoprotein, and of translating that genomic sequence into its corresponding protein sequence can also be used.

An antigenic domain that is about 10 to about 40 amino acid residues long is then selected from the overall protein sequence. A synthetic polypeptide substantially corresponding to the selected 10 to 40 amino acid residue long domain is then synthesized by usual solid-phase synthetic methods. [Houghten et al., Int. J. Pept. Prot. Res., 16:337-340 (1981); Merrifield, J. Am. Chem. Soc., 85:2149-2154 (1963)]. Longer polypeptide sequences can be synthesized and be used. However, the probability of finding a desired homologous sequence of greater than about 40 consecutive amino acid residues in t unrelated oncoprotein molecules is virtually nonexistent, and consequently, antibodies raised to a longer synthetic polypeptide may not immunoreact well with a second oncoprotein. Thus, synthetic polypeptides containing about 40 amino acid residues, or more, are most useful for inducing antibodies that do not cross-react with a domain of a second oncoprotein. Such antibodies immunoreact with the oncoprotein to whose determinant domain they were raised and inhibit a reaction at an active site of that oncoprotein. In more preferred practice, the synthetic polypeptide contains a sequence of about 10 to about 25 amino acid residues.

It is likely that hydrophilic domains of the oncoprotein are exposed on the cell liquid-contacting portion of the native molecule, thereby permitting good contact between an induced antibody and the oncoprotein. It is therefore preferable that the oncoprotein polypeptide sequence selected for synthesis contain hydrophilic amino acid residues such as Arg, Lys, Asp, Glu, and His. Selection of a hydrophilic oncoprotein polypeptide domain also provides a synthetic polypeptide that is water-soluble. Such solubility is desirable.

It is also preferable to select a determinant domain sequence between hydrophobic residues and/or proline residues. Those residues are often buried within the native oncoprotein while the selected, hydrophilic determinant domain therebetween is exposed.

A determinant domain that is less than about 200 amino acid residues away from an oncoprotein active site such as tyrosine-419 of the pp60$^{src}$ oncoprotein is preferably selected for synthesis so that an immunoglobulin molecule can bind to the determinant domain of the oncoprotein and in so binding, physically block the active site. In more preferred practice, the domain selected to be synthesized is less than about 150 amino acid residues from the oncoprotein active site. The oncoprotein determinant is most preferably less than about 100 amino acid residues from the oncoprotein active site. Inasmuch as an oncoprotein can contain 1000 or more amino acid residues, the preferred oncogenic domain is "vicinal" to the oncoprotein active site, as that term is used herein and in the appended claims.

It is frequently convenient to add one or more additional amino acids to the amino- or carboxy-termini of the synthetic polypeptide to assist in binding the synthetic polypeptide to a carrier to form a conjugate. Cysteine residues added at the carboxy-terminus of the synthetic polypeptide have been found to be particularly useful for forming conjugates via disulfide bonds, but other methods well known in the art for preparing conjugates may be used. Exemplary binding procedures include the use of dialdehydes such as glutaraldehyde and the like, or the use of carbodiimide technology as in the use of a water-soluble carbodiimide, e.g. 1-ethyl-3-(3-dimethylaminopropyl) carbodiimide.

Useful carriers are well known in the art and are generally proteins themselves. Exemplary of such carriers are keyhole limpet hemocyanin (KLH), albumins such as bovine serum albumin or human serum albumin (BSA or HSA, respectively), red blood cells such as sheep erythrocytes (SRBC), tetanus toxoid, as well as polyamino acids such as poly(D-lysine:D-glutamic acid), and the like.

As is also well known in the art, it is often beneficial to bind the synthetic polypeptide to its carrier by means of an intermediate, linking group. As noted above, gluteraldehyde is one such linking group, while when cysteine is utilized, the intermediate linking group is preferably a m-maleimidobenzoyl N-hydroxysuccinimide ester (MBS). MBS is typically first added to the carrier by an ester-amide interchange reaction, followed by addition of a blocked mercapto group such as thioacetic acid across the maleimido-double bond. Cleavage of the blocking group follows, and then the disulfide bond is formed between the deblocked linking group mercaptan and the mercaptan of the added cysteine residue of the synthetic polypeptide.

B.  Immunization and Antibody Response to Selected Synthetic Polypeptides

The synthesized polypeptide(s) or its conjugate(s) is used for immunization of test animals such as rabbits according to the usual procedures discussed hereinafter. Test bleeds are then taken from the animals starting on about the fifth week after the first immunization and serum samples from the test bleeds are examined for the development of anti-synthetic polypeptide antibody titer by standard enzyme-linked immunosorbent assay (ELISA) procedures. ELISA determinations are discussed in Maggio, Enzyme-Immunoassay, CRC Press, Boca Raton, Fl., 1981, whose disclosures are incorporated herein by reference.

For example, a constant amount of about 5 to about 15 picomoles of the unconjugated synthetic polypeptide is immobilized in each well of a plastic microtiter plate. The free plastic surface of the plate is blocked from nonspecific attachment of antibody molecules by a protein. Where a polypeptide conjugate is used, a protein not used as the carrier protein in the immunization procedure is utilized as the blocking protein.

Serial dilutions of test antisera are allowed to interact with the predetermined, constant amount of synthetic polypeptide present in each of a series of wells. The amount of antipeptide antibody present in the dilutions of the test bleed serum is determined by measuring the amount of an enzyme conjugate of a second antibody directed against the immunoglobulins of the test animal immunized, e.g., rabbit, is bound to the polypeptide - antipolypeptide complex. This admixture of the plastic-bound synthetic polypeptide, antibody to that synthetic polypeptide, and antibodies raised to the first antibodies results in a complex which can be schematically depicted as:

bound polypeptide/rabbit antipolypeptide antibody/goat anti-rabbit immunoglobulin-enzyme

for the situation where a rabbit is immunized to produce the first antibody and goat anti-rabbit antibodies comprise the second antibody.

Exemplary useful enzymes that can be bound to the second antibody include horseradish peroxidase, alkaline phosphatase and glucose oxidase.

Since an excess of synthetic polypeptide is immobilized in the wells, the amount of enzyme bound in each well directly reflects the amount of antibody molecules in the antipolypeptide serum solution in the respective wells. The results are plotted as enzyme activity versus serum dilution. A typical plot for a specific antiserum raised against a dodecapeptide is illustrated in Figure 4.

A similar determination can thereafter be made as to which of the synthetic polypeptides or conjugates induced production of antibodies which also recognize and immunoreact with the oncoprotein, as by immunoprecipitation. The antibodies so produced are also tested for their abilities to inhibit a reaction at an oncoprotein active site and for their cross-reactivity with oncoproteins from other viral strains and/or neoplastic cells.

Further amounts of the antibodies to that synthetic polypeptide(s) or its conjugate are raised in antisera, and the antibody-containing antisera are collected once it is determined which synthetic polypeptide(s) or conjugate elicits a high antibody titer upon immunization. Particularly preferred antibodies (1) immunoreact with the oncoprotein whose antigenic domain amino acid residue sequence corresponds to the synthetic polypeptide, (2) inhibit a reaction at an oncoprotein active site and (3) cross-react with an oncoprotein from another viral strain or neoplastic cells.

Idiotype-containing relatively smaller portions of antibody molecules are also useful herein. Such antibody portions include the well known Fab and F(ab')$_2$ fragment portions. These antibody portions can be prepared by proteolytic cleavage of the antibody molecule by the enzymes papain and pepsin, respectively, followed by purification using well known techniques.

The antibodies of this invention, along with their smaller portions that include the antibody idiotypic regions such as the Fab and F(ab')$_2$ fragments and the like, are collectively referred to herein as idiotype-containing polyamides. The idiotype-containing polyamides recognize and bind to the antigenic determinant domain of the oncoprotein to whose amino acid residue sequence they were raised (first oncoprotein). Idiotype-containing polyamides are raised to the amino acid residue sequence of the determinant domain of the first oncoprotein by usual immunization techniques using a synthetic polypeptide or its conjugate of this invention as the immunizing antigen.

The idiotype-containing polyamides can also immunoreact with a second oncoprotein having an antigenic determinant domain homologous to the above recited domain of the first oncoprotein. Some idiotype-containing polyamides such as intact or substantially intact antibodies can also inhibit a reaction at an active site of an oncoprotein, depending, inter alia, upon the length of the idiotype-containing polyamide and the proximity of the oncoprotein domain bound by the idiotype-containing polyamide to an active site of that oncoprotein.

C.   Inhibition of Reactions at an Oncoprotein Active Site

Oncoproteins are reactive with other components present in the cell via the oncoprotein active site. These reactions include catalysis such as phosphotransfer. Antibodies raised to the present synthetic polypeptides bind to the oncoprotein and inhibit a reaction at the oncoprotein active site.

Oncoproteins from at least six oncogenes (v-src, v-fps, v-yes, v-ros, v-abl, and v-fes) have been found to catalyze phosphotransfer reactions. Inhibition of the phosphotransfer reaction by antibodies to the synthetic polypeptide or its conjugate provides an exemplary demonstration of the more general phenomenon of inhibition of a reaction at an active site of an oncoprotein that is characterisic of the antibodies of this invention.

Thus, immune complexes may be prepared from (a) the relatively longer idiotype-containing polyamides or more preferred intact antibodies raised to a synthetic polypeptide or conjugate of this invention combined with (b) unlabeled cell lysates from cells containing the oncoprotein whose determinant domain amino acid residue sequence was synthesized in the synthetic polypeptide. After suitable purification such as washing of the immune complex, it is incubated in an appropriate buffer containing gamma-$^{32}$P-ATP for a predetermined period of time. The complex is thereafter washed again, dissociated and then subjected to gel electrophoresis using usual techniques. The gels are dried and autoradiograms are then prepared.

Results of an inhibition of a reaction at an active site of an oncoprotein are illustrated in Figure 2A. The photographs of autoradiograms shown in Figure 2A illustrate that immune complexes formed with antisera containing antibodies raised to the 12 and 30 residue polypeptides whose syntheses are discussed hereafter did not transfer a phosphate group from the labeled ATP molecule to the v-fes oncoprotein. The results shown in lanes 8 and 15 of Figure 2A illustrate that immune complexes formed using anti-gag antiserum and either ST-FeSV or GA-FeSV transformed cell lysates phosphorylated the gag-fes polyprotein.

The results in Figure 2A also point out the importance of selecting a determinant domain of the oncoprotein as being vicinal to the active site. Thus, the ST-FeSV transformed FeLV producer mink cells (lanes 5 through 8) and GA-FeSV transformed nonproducer mink cells (lanes 12 through 15) produced a fused gag-fes polyprotein. Phosphotransfer occurred when anti-gag antiserum was used as the source of antibodies (lanes 8 and 15, respectively) while antibodies to the fes-oncoprotein portion of the fused polyprotein raised by immunization with the polypeptide conjugates of this invention inhibited the phosphorylation reaction (lanes 6, 7, 13 and 14).

These results show that antibodies raised to synthetic polypeptides from a portion of the oncoprotein vicinal to the active site inhibited the phosphortransfer reaction while antibodies raised to the gag portion of the fused protein bound to determinant domains too far away from the reaction-related active site to inhibit the phosphotransfer reaction.

D.   Immunoprecipitation of Oncoproteins

The oncoprotein encoded by the viral oncogene is stably expressed in transformed, cultured cells. Thus, normal cultured cells and cells transformed by the oncovirus that encodes the oncoprotein whose determinant domain substantially corresponds to the amino acid sequence of the synthetic polypeptide e.g., feline sarcoma virus, are metabolically labeled with a radioactive amino acid such as methionine or leucine. The cells are lysed open and the lysate is contacted with the above-prepared anti-synthetic polypeptide serum.

Proteins from the cell lysates bound to the anti-synthetic polypeptide antibodies in the serum are then separated by the ability of the antibodies to bind to the bacteria Staphylococcus A. The bound protein molecules thus isolated from normal and transformed cells are then dissociated from the complex and are resolved by electrophoretic techniques that separate protein molecules based upon their sizes.

Autoradiography of the electrophoresis gels identifies the proteins from the lysed cells which bind to the anti-synthetic polypeptide antibodies. The results of a typical determination are shown in Figure 1.

As can be seen from examination of Figure 1, the antibodies from the anti-synthetic polypeptide sera bind large quantities of specific proteins from transformed cells (85 Kd protein in lanes 7 and 8 and a 108 Kd protein in lane 13), and does not substantially bind proteins from the normal parent cell lines (lanes 2 and 3). This type of immunoprecipitation- electrophoresis study is done routinely for every anti-synthetic polypeptide antiserum using cells transformed with the viruses from which the oncogene was identified.

The results illustrated in Figure 1 point out the specificity of antibodies elicited to synthetic polypeptides corresponding to a determinant domain of the oncoprotein that is vicinal to but exclusive of an active site of the oncoprotein. Thus, substantially only the oncoproteins were immunoprecipitated with the anti-synthetic polypeptide antibodies from transformed cells while substantially nothing was immunoprecipitated from the nontransformed cells. This is in contrast to the results reported in the above paper by Wong and Goldberg wherein the antibodies raised to the active site of the oncoprotein immunoprecipitated substantial amounts of proteins in both transformed and nontransformed cells.

Because of the determinant domain specificity of the antibodies prepared according to this invention, the above immunoprecipitation-electrophoresis technique can also be utilized to identify oncoproteins that have related or homologous determinant domains but are produced in cells transformed by a different oncovirus or are produced in neoplastic cells from animals such as humans in which neoplasticity may not have been induced by a known viral infection. Thus, the results illustrated in Figure 3 show that antibodies raised to a conjugate containing the dodecapeptide discussed hereinafter (Section III) that corresponds to a determinant domain of the oncoprotein encoded by the v-fes gene also precipitate an oncoprotein encoded by the v-fps gene, the oncogene of the Fujinami strain of avian sarcoma virus. Figure 5 also illustrates that the same antibodies to that dodecapeptide conjugate immunoprecipitate a 40 Kd oncoprotein in cell lines derived from various human tumors.

The transcription of v-onc-related nucleic acids in human tumors is illustrated by the work of Eva et al., Nature, 295, 116-119 (1982). Those workers detected polyadenylated RNA transcripts related to the sis, myc, abl and bas oncogenes in almost all of the tumor cells analyzed. However, they also found such RNA transcripts in normal human fibroblasts.

## III.   Illustrative Examples of the Invention

Various aspects of the present invention are illustrated in this section with regard to the oncoprotein encoded by the oncogene v-fes of feline sarcoma virus.   It is to be understood however that the principles illustrated herein are general and are not to be construed as being limited soley to the oncogene and oncoproteins described herein.  Details of experimental procedures are provided in the Materials and Method section (IV) that follows this discussion.

Two synthetic polypeptides that substantially correspond to an antigenic domain near the carboxy-terminus of the oncoprotein encoded by the v-fes gene were prepared.   Sequences of the two synthetic polypeptides, denominated polypeptide I and polypeptide II, are shown below, written from left to right in the direction from amino-terminus to carboxy-terminus:

Polypeptide I:

SerAspValTrpSerPheGlyIleLeuLeuTrpGluThr
PhePheLeuGlyAlaSerProTyrProAsnLeuSerAsnGlnGlnThrArgCys

Polypeptide II:

SerProTyrProAsnLeuSerAsnGlnGlnThrArgCys.

Using the well known single letter abbreviations for amino acid residues, the 30 residue polypeptide I has the sequence, written from left to right in the direction from amino-terminus to carboxy-terminus:

S-D-V-W-S-F-G-I-L-L-W-E-T-F-F-L-G-A-S-P-Y-P-N-L-S-N-Q-Q-T-R-C

and the dodecapeptide, polypeptide II, has the sequence written in the same manner:

S-P-Y-P-N-L-S-N-Q-Q-T-R-C.

The underlined cysteine (Cys or C) residue at the carboxy-terminus of each of the above polypeptides is not present in the oncoprotein amino acid residue sequence, but was added to assist in conjugation via a disulfide bond to the carrier.

For ease in understanding, the correspondence between the three letter and one letter codes for the twenty usually found amino acids is given below:

| Ala A | Ile I | Arg R |
| Cys C | Lys K | Ser S |
| Asp D | Leu L | Thr T |
| Glu E | Met M | Val V |
| Phe F | Asn N | Trp W |
| Gly G | Pro P | Tyr Y |
| His H | Gln Q | |

The oncoprotein determinant domain represented by the above 30-residue sequence has several interesting features. First, it is located vicinally to the active site that contains the tyrosine residue that is phosphorylated in reactions catalyzed in an immune complex containing the gag-fes polyprotein. Second, the domain is substantially identical between the oncoproteins encoded by the v-fes oncogene of the Snyder-Theilen and Gardner-Arnstein strains of feline sarcoma viruses. Third, it contains extensive homologies to a domain in the oncogene v-fps the Fujinami of avian sarcoma virus. [Hampe et al., Cell, 30, 775-785 (1982); and Shibuya and Hanafusa, Cell, 30, 787-795 (1982).]

Homologous regions of the oncoproteins encoded by the v-fes and v-fps oncogenes are illustrated below, from left to right in the direction from amino-terminus to carboxy-terminus, with brackets surrounding the dodecapeptide of polypeptide II as well as its homologous region in the v-fps oncoprotein:

       v-fes:        ...F-S-L-G-A-[S-P-Y-P-N-L-S-N-Q-Q-T-R-]E-F-V-E...

       v-fps:        ...F-S-L-G-A-[V-P-Y-A-N-L-S-N-Q-Q-T-R-]E-L-V-E...

The homologous regions of dodecapeptides encoded by the v-fes and v-fps genes can be written together from left to right in the direction from amino-terminus to carboxy-terminus as:

      S(V)-P-Y-P(A)-N-L-S-N-Q-Q-T-R

wherein each of the amino acid residues in parentheses is an alternative to the immediately preceding amino acid residue.

It is noted that for the immediately above amino acid sequence and those disclosed hereinafter, each alternative amino acid residue need not be replaced in an individual synthetic polypeptide of this invention. Rather, the parenthesized amino acid residues are independently alternative. Thus, the above sequence including parentheses encompasses the amino acid residue sequence of the four dodecapeptides shown below, written in the same manner:

      S-P-Y-P-N-L-S-N-Q-Q-T-R,

      V-P-Y-A-N-L-S-N-Q-Q-T-R,

      S-P-Y-A-N-L-S-N-Q-Q-T-R, and

      V-P-Y-P-N-L-S-N-Q-Q-T-R.

Each of polypeptides I and II was coupled to KLH and used to immunize rabbits as described in the Materials and Methods section. The development of antibody titer against the two peptides was monitored by the ELISA procedure described in that section also.

Antibody titers were detectable within five weeks after immunization. These antisera showed a considerable level of cross-reactivity as can be seen from the data in Table 1 hereinbelow.

A titer of greater than 1:10,000 was obained within seven weeks using the conjugate of synthetic polypeptide II. Using the longer synthetic polypeptide, polypeptide I, a titer of approximately 1:5000 was observed within seven weeks. Antisera from animals immunized with synthetic polypeptide I showed only a very weak cross-reactivity to synthetic polypeptide II (Table 1).

It is noted that once immunized with KLH-coupled conjugate, the synthetic polypeptide I was sufficiently immunogenic to show a secondary immune response. The titers of anti-synthetic polypeptide antibodies were stable in the immunized rabbits for six to eight weeks even if the animals were not boosted.

## Table 1

### Antibody Titer of Immunized Rabbit Sera Against Synthetic Peptides

| Immunizing Antigen Conjugate | Antibody Titer[1] Against | |
|---|---|---|
| | Polypeptide I | Polypeptide II |
| Polypeptide I | | |
| Animal 1 | 1:5,600 | 1:250 |
| Animal 2[2] | 1:4,000 | 1:200 |
| Polypeptide II | | |
| Animal 1 | 1:2,000 | >1:10,000 |
| Animal 2 | 1:1,800 | >1:10,000 |

---

[1] ELISA assay with immobilized synthetic polypeptide was performed and the results were plotted as described in the Materials and Methods section. The antiserum dilution required to attain one-half maximal color intensity is expressed as the "Antibody Titer" in this table.

[2] This animal received KLH-polypeptide I conjugate only for primary immunization. Subsequent injections used free polypeptide I.

Nucleotide sequence analysis of the Gardner-Arnstein (GA) and the Snyder-Theilen (ST) strains of the feline sarcoma (FeSV) predicts that the v-fes gene of GA-FeSV can encode a 957 amino acid residue protein while that of the ST-FeSV has a coding capacity of 774 amino acids (Hampe et al., supra). Immunoprecipitation studies by others using antisera directed aginst the determinants to proteins encoded by feline leukemia virsus (FeLV-GAG, e.g., anti-FeLVp15 or anti-FeLVp27), showed the detection of a 108 Kd protein from GA-FeSV transformed cells and a 85 Kd protein from ST-FeSV transformed cells. [See Barbacid et al., Proc. Natl. Acad. Sci. USA, 77, 5158-5162 (1980); Ruscetti et al., J. Virol., 35, 259-264 (1980) and Van de Ven, et al., Virology, 101, 185-197 (1980)]. These proteins were also detected by the immunoprecipitation of metabolically labeled proteins using antisera from rats bearing FeSV-induced tumors. The presence of the 85 Kd protein in ST-FeSV transformed cells and the presence of the 108 Kd protein in GA-FeSV transformed cells are in good agreement with the molecular weights of the protein predicted by a combined nucleotide sequence of the gag and fes genes with the corresponding viruses.

The antisera against both of the synthetic polypeptides were used to immunoprecipitate metabolically labeled proteins from GA- and ST-FeSV transformed mink cells. An SDS-PAGE analysis of the immunoprecipitates is shown in Figure 1.

By choosing polypeptides for synthesis that correspond to a determinant domain of the fes-oncoprotein that is vicinal to but exclusive of an active site of the oncoprotein, no specific radioactive band was immunoprecipitated by any of the immune sera from normal mink cells (lanes 1-5). The antiserum (5016) directed against synthetic polypeptide II recognized the gag-fes polyprotein of approximately 108 Kd in the GA-FeSV transformed nonproducer cells (lane 13) and the 85 Kd polyprotein in the ST-FeSV transformed producer cells (lane 8) This serum did not precipitate the gag polyprotein either in the producer transformed cells (lane 8) or from FeLV-infected, nontransformed cells (lane 17).

The antiserum (5014) against synthetic polypeptide I, the larger synthetic polypeptide, immunoprecipitated the ST-FeSV polyprotein (lane 7) but failed to recognize the GA-FeSV polyprotein (lane 12). The anti-FeLVp27 antiserum recognized an approximately 108 Kd protein in the GA-FeSV transformed nonproducer cell line, 64F3C17 (lane 13). As expected in FeLV-infected nontransformed mink cells, the anti-p27 serum precipitated the 65 Kd gag polyprotein, Pr65gag (lane 18). In the ST-FeSV transformed FeLV-producer mink cell line, MSTF, the antiserum precipitated both the 65 Kd polyprotein and the ST-FeSV specific gag-fes polyprotein of approximately 85 Kd (lane 10). An antiserum against FeLV-15, like the anti-p27, recognized both the gag-fes polyprotein and the gag polyprotein from ST-FeSV transformed producer cells (lane 9).

A comparison of the relative immunoprecipitability of the gag-fes polyprotein from the two transformed cell lines using anti-gag antisera and the antisera directed against synthetic polypeptide II revealed a difference. The anti-synthetic polypeptide sera appeared to recognize the ST-FeSV polyprotein more efficiently than the GA-FeSV polyprotein.

It is also noted that the anti-synthetic polypeptide II recognized a minor band at approximately 100 Kd in all cells including the normal mink cell. The origin of this protein is not clear at this time. However, the immunoprecipitation of only one minor protein band in addition to the oncoprotein by the antisera to the synthetic polypeptides of the present invention is a vast improvement over the results reported by Wong and Goldberg. Moreover, there have been indications that the c-fes gene encodes for an approximately 95 Kd protein in some normal mammalian and avian cells [Barbacid et al., Proc. Natl. Acad. Sci. U.S.A., 77, 5158-5162 (1980) and Mathey-Prevot et al., Cell, 28, 897-906 (1928)]. The observed minor band may therefore be the product encoded by the c-fes gene that is normally present.

These immunoprecipitation studies provide conclusive evidence that the v-fes gene product is expressed in the form of an 85 Kd protein in the ST-FeSV transformed cells and as a 108 Kd protein in the GA-FeSV transformed cells. Prior to the availability of the antisera produced in accordance with this invention, these proteins have been identified only indirectly by immunoprecipitation with anti-FeLV-gag antisera from FeSV transformed cells.

Antisera raised against the amino-terminus determinants (p15) of the GAG gene and the synthetic polypeptide antibody-containing sera directed toward polypeptides derived from the 3'-end of the fes coding sequence were shown to recognize the candidate polyprotein molecules from two related but distinct FeSV isolates. The data thus also confirm cotranslations of the GAG and the fes genes from the reading frame predicted by the nucleotide sequences of GA and ST-strains of FeSV. The 85 Kd protein of the ST-FeSV and the 108 Kd protein of GA-FeSV can thus be justifiably termed $p85^{gag-fes(ST)}$ and $p108^{gag-fes(GA)}$, respectively.

The short dodecapeptide, synthetic polypeptide II, has produced high titer antisera. The domain on the oncoprotein corresponding to the synthetic polypeptide is located vicinal to the active site of tyrosine phosphorylation of the v-fes protein, and complexing with anti-synthetic polypeptide antibodies which bind to the determinant domain vicinal to that active site inhibited the autophosphorylation reaction of that oncoprotein as is discussed further hereinafter.

Others have shown that the oncoprotein of v-fes has a tyrosine kinase activity associated with it. [See Hunter et al., Proc. Natl. Acad. Sci. USA, 77, 1311-1315 (1980); Van de Ven et al., supra; and Reynolds et al., J. Virol., 37, 643-653 (1981).] Immune complexes formed between the gag-fes polyprotein and antisera directed against FeLV-gag determinants have been found to catalyze an autophosphorylation reaction leading to the transfer of $^{32}P$- radioactivity from gamma-$^{32}P$-labeled ATP to a unique tyrosine residue on the v-fes oncoprotein. [Hampe et al., supra; and Neil et al., Virology, 109, 223-228 (1981).]

Immune complexes were formed as described in the Materials and Methods section from GA-FeSV transformed cell lysates using anti-synthetic polypeptide antiserum and the anti-gag antiserum, and were tested for their ability to catalyze phosphorylation in vitro. The results of these phosphotransfer reactions are illustrated in Figure 2A.

Immune complexes using anti-gag antiserum with either the ST-FeSV or the GA-FeSV transformed cell lysate catalyzed phosphorylation of the respective gag-fes polyproteins (lanes 8 and 15, respectively). In contrast, the immune complexes formed using antisera raised to either of the synthetic polypeptides I and II failed to catalyze a phosphorylation reaction (lane 6, 7, 13, and 14). This was the case even though under the conditions used for the formation of the immune complexes, the antisera against peptide II clearly precipitated the gag-fes polyprotein in amounts comparable to that obtained with the anti-gag serum.

The reason for these results is believed to lie in the fact that the oncoprotein domain corresponding to synthetic polypeptide II is located vicinal to (approximately 45 amino acid residues away from) the active tyrosine-phosphorylation site on the v-fes oncoprotein as determined by Blomberg et al., J. Virol., 38, 886-894 (1981). It is thus believed that the anti-synthetic polypeptide antibody binds to this oncoprotein vicinally to the site of phosphorylation and physically blocks the phosphotransfer reaction while the anti-gag antibody binds distal to this active site and permits phosphorylation to occur.

This hypothesis of the different binding sites for anti-gag and anti-synthetic polypeptide antibodies was examined by use of aliquots of a lysate from ST-FeSV transformed cells which were incubated in parallel with differing ratios of anti-gag to anti-synthetic polypeptide antibody while keeping the total amount of antibody constant. Immune complex kinase assays were carried out with the differnet immunoprecipitates.

The results of this determination are illustrated in Figure 2B and show that as the ratio of anti-synthetic polypeptide antibodies to anti-gag antibodies increased, the extent of phosphorylation of the approximately 85 Kd polyprotein was reduced. At a ratio of anti-sythetic polypeptide antisera to anti-gag antisera of 25:1, tyrosine phosphorylation of the 85 Kd polyprotein was no longer detectable. In a parallel experiment, similar mixtures of antibodies were found to be in saturating concentrations for the amount of lysates used and thus precipitated virtually identical amounts of the ST-FeSV polyprotein for metabolically labeled cells.

This type of neutralization of phosphorylation of the polyprotein with increasing amounts of anti-synthetic polypeptide serum is consistent with the above hypothesis that the enzymatic phosphotransfer is blocked by the binding of the anti-synthetic polypeptide immunoglobulin molecules vicinal to the active site of phosphorylation of the v-fes oncoprotein.

The ability of the anti-v-fes synthetic polypeptide serum to recognize the v-fps oncoprotein from stable transformed NRK cells was determined. The results of immunoprecipiation using metabolically labeled cell proteins and antibodies against synthetic polypeptide I and against synthetic polypeptide II are shown in Figure 3A, while the experimental procedures are discussed in the Materials and Methods section. The antisera against synthetic polypeptide II immunoprecipitated the 130 Kd gag-fps oncoprotein from the transformed NRK cells (lane 2). Antiserum against the larger polypeptide, synthetic polypeptide I, failed to recognize the protein (lane 3).

Since the v-fes and v-fps are highly related and both contain similar tyrosine kinase activities [Barbacid et al., supra; and Beemon, Cell, 24, 145-153 (1981)], immune complexes were prepared from lysates of FSV-transformed and normal NRK cell lines using normal rabbit serum, tumor bearing rabbit (TBR) serum from Rous sarcoma virus (RSV) that cross-reacts with the Fujinami-fps protein, or the antiserum directed against synthetic polypeptide II. The complexes were incubated with gamma-$^{32}$P-ATP and the products of incubation were analyzed on discontinuous SDS gel electrophoresis. These results are illustrated in Figure 3B.

The immunoprecipitates prepared using antisynthetic polypeptide serum and FSV-transformed cells did not phosphorylate the _fps_ p130 oncoprotein or the Ig H-chain (lane 3). Immune complexes prepared with the TBR serum did catalyze the phosphorylation of the viral p130 oncoprotein and the Ig H-chain (lane 2). With normal NRK cell lysates, the anti-synthetic polypeptide antibody immunoprecipitate again did not catalyze any phosphorylation (lane 6), while the TBR immunoprecipitates did phosphorylate the Ig H-chains at a very low level (lane 5), presumably catalyzed by the endogenous cellular _c-src_ kinase.

Only a limited number of amino acid residues are shared between the _v-fes_ and the _v-src_ oncoproteins. [Hampe et al. supra; Shibuya et al., supra and Czernilofsky et al., _Nature_, 287, 198-203 (1980).] There is thus little homology in antigenic domains between these oncoproteins. It was therefore not surprising that antibodies raised to polypeptide II did not immunoprecipitate the pp60[src] oncoprotein from RSV transformed rat cells.

The results obtained with the feline sarcoma virus (FeSV) transformed cells, thus, again show that the binding of the anti-synthetic polypeptide antibodies vicinal to the active tyrosine phosphorylation site of the oncoprotein inhibits the tyrosine kinase activity. In this case, both the autophosphorylation and the substrate (Ig H-chain) phosphorylation reactions were inhibited by the anti-synthetic polypeptide antibody molecules. Thus, a defined antiserum against chemically synthesized polypeptides corresponding to a specific domain of an oncoprotein encoded by a viral oncogene has permitted demonstration of cross-reactivity against the product(s) of another retroviral oncogene. Indeed, one syntehtic polypeptide which is virtually identical between the oncoproteins coded for by the v-fes and the v-fps genes has provided antisera that will immunoprecipitate products from both transformed cells.

In addition, the protein kinase activity of both of the v-fes and v-fps oncogene products was inhibited when antibodies bound to domains vicinal to the active sites. Namely, neither the autophosphorylation of the v-fes encoded oncoprotein, nor the autophosphorylation or immunoglubulin phosphorylation-catalyzed by the v-fps oncoprotein were observed when those proteins were bound to antisera raised to the synthetic polypeptide II. These data also suggest that the domains around the active site in these two related proteins have similar conformations.

IV. Methods and Materials

    A.    Cells and Viruses

The normal mink cell line CCL64 (American Type Culture Collection) was used to obtain a nonproducer cell line transformed with the Gardner-Arnstein strain of feline sarcoma virus (FeSV), 64F3C17 and a nontransformed cell line chronically producing feline leukemia virus (FeLV) subgroup B. The transformed producer culture, MSTF, produces high levels of the Snyder-Theilen strain of FeSV with a subgroup B helper [Robbins et al., Virology, 97, 1-11 (1979)]. The Fujinami sarcoma virus transformed rat cell line, [Hanafusa et al., Proc. Natl. Acad. Sci. USA, 77, 3009-3013 (1980)] was derived from the normal rat kidney line, NRK [Duc-Nguyen et al., J. Bacteriol, 92, 1133-1140 (1966)]. Another nonproducer line of transformed NRK line containing the Rous strain of avian sarcoma virus (RSV) was obtained from Dr. Peter Vogt of the University of Southern California.

All cells were maintained in Dulbecco's modification of Eagle's medium with high glucose (4500 milligrams/liter) supplemented with 10% fetal calf serum.

B. Polypeptide Synthesis

The polypeptides were synthesized by the solid phase method [Merrifield, J. Am. Chem. Soc., 85, 2149-2154 (1980)] using a cysteine resin according to previously described methodology [Houghten et al., Int. J. Pep. Prot. Res., 16, 311-320 (1980)]. The individual amino acids had their side chains protected as follows: Arg-tosyl, Ser, Thr, Glu and Asp-O-benzyl; Tyr-O-bromobenzyloxy carbamyl; Trp-N-formyl. The N-formyl group on the Trp residues was removed after cleavage of the peptide from the resin support by treatment with 1.0 molar ammonium bicarbonate at a peptide concentration of 1.0 milligram/milliliter for 16 hours at the room temperature. [Yamashiro et al., J. Org. Chem., 38, 2594-2597 (1973).] The efficiency of coupling at each step was monitored with ninhydrin [Kaiser, Anal. Biochem., 34, 595-598 (1980)] and/or picric acid [Gisin, Anal. Chem. Aeta, 58, 248-249 (1972)] and was greater than 99% in all cases. An amino acid analysis of the completed peptide gave the correct values (±5% predicted values).

C. Polypeptide Conjugation, Immunization and Estimation of Antiserum Titers

Each peptide was reduced with dithiothreitol immediately prior to its conjugation with freshly activated keyhole limpet hemocyanin (Calbiochem-Behring, La Jolla, CA) using previously described procedures [Sutcliffe et al., supra].
Adult male New Zealand white rabbits were each
injected with about 200 micrograms of peptide-KLH
conjugate in complete Freund's adjuvant (a
physiologically tolerable vehicle) on day 1; they
were boosted with 200 micrograms of the conjugate in
incomplete Freund's adjuvant on day 14 and again with
100 micrograms conjugate mixed with aluminum
hydroxide on day 21. Test bleeds were collected
weekly following the last immunization. Preimmune
sera and test bleeds were all partially fractionated
by adding ammonium sulfate to 40% final saturation,
and the precipitated protein dissolved in the
starting serum volume of PBS was used as the source
of antibodies in all experiments.

Antibody titers against the peptides were
measured by an enzyme-linked immunosorbent assay
(ELISA) using 5 picomoles of peptide immobilized in
each well of 96-well microtiter plates (Falcon
Plastics); a glucose oxidase conjugate of affinity
purified goat anti-rabbit immunoglobulins was used as
the assay enzyme [Niman et al., Proc. Natl. Acad.
Sci. USA, 77, 4524-4528 (1980)]. Following
development of assay incubations, the absorbence was
plotted against the serial dilutions for each serum.
The antiserum dilution giving one-half the maximal
color at the end of the linear range of the plot was
taken as the titer of the antiserum.

D. Metabolic Labeling, Immunoprecipitations
and Immune Complex Kinase Assays

Cells were grown to approximately 80%
confluency in 6 well Falcon tissue culture plates
(Falcon 3046) in normal serum-supplemented DMEM. The
monolayer was rinsed with 1 milliliter of
methionine-free DMEM and then incubated for 2 hours
in methionine free medium.

The cells were then labeled for 2-4 hours with 50 microCuries of $^{35}$S-methionine (about 1000 Curies/millimole, New England Nuclear, Boston, MA) in methionine-free medium using 0.5 milliliters per well. At the end of the labeling period, the labeling media were removed and the cell monolayer was lysed with 0.4 milliliters of IPB (immunoprecipitation buffer which contains 20 millimolar Tris-HCl pH 7.2, 1% NP40, 0.5% sodium deoxycholate, 0.1% SDS, 2 millimolar sodium azide, 2 millimolar phenylmethylsulfonyl fluoride, 2% aprotinin and 1 molar sodium chloride).

The lysate was clarified at 12,000 x gravity in a microfuge for 15-20 minutes at 4°C. Each lysate was then precleared by incubating for 2 hours at 4°C. with 100 microliters of preimmune rabbit sera. The immune complexes were removed by incubating with 100 microliters of formeldehyde-fixed Staphylococcus A. available commercially under the designation Pansorbin from Calbiochem-Behring, La Jolla, CA.

Aliquots of the precleared lysates were incubated with desired quantities of various test sera at 4°C. for 2 hours. Immune complexes were collected using Pansorbin, and were washed three times with IPB followed by one wash with 0.1 molar Tris-HCl (pH 8.5) containing 0.5 molar lithium chloride and one final wash with phosphate-buffered saline. The washed immune complexes were dissolved directly in Laemmli sample bufer (Laemmli, supra), boiled and clarified by centrifugation. The supernatants were analyzed on discontinuous acrylamide gel electrophoresis (Laemmli, supra). The gels were treated with Enhance (New England Nuclear, Boston, MA), then dried and exposed to Kodak XRP-5 film.

For the immune complex kinase assays, immune complexes were made from unlabeled cell lysates as described above. The final complex was washed once in 10 millimolar Tris-HCl (pH 7.4) containing 5 millimolar magnesium chloride.

Each washed pellet was then incubated in 25 microliters of the above Tris-MgCl$_2$ buffer containing 1-2 microCuries of gamma-$^{32}$P-ATP (about 3000 Curies/millimole) for 20 minutes at 22°C. At the end of the incubation, the pellets were washed once with PBS, then dissociated with Laemmli SDS-PAGE sample buffer and electrophoresed as above. The gels were dried and exposed to Kodak XRP-5 films.

V. Host Protection

Three young adult cats were immunized with the polypeptide II-KLH conjugate described above (Sections III and IV). The immunizations were carried out with two doses each of 0.5 milligrams of conjugate in incomplete Freund's adjuvant at two week intervals. Three parallel cats were left as untreated controls.

One month after the first immunization, each of the six cats was challenged by injection of approximately 1 x 10$^6$ feline tumor cells producing relatively high levels of both FeSV and FeLV. Antibody titers using the above ELISA method were determined for each cat prior to challenge. The results of the immunizations and protection of the immunized cats by the polypeptide II-conjugate are illustrated in Table 2, below.

Table 2

Comparative Protection

Day of Post Challenge

| Cat. No. | Antibody Titer | Tumor Appearance |
|---|---|---|
| Immunized | | |
| DZ4 | 1:3600 | 15 |
| 214 | 1:1500 | 4-7 |
| 232 | 1:1500 | 11 |
| Control | | |
| IB2 | less than 1:50 | 4 |
| 253 | less than 1:50 | 4 |
| 271 | less than 1:50 | 7 |

As can be seen from the above results, the immunized cats were protected relative to the control cats. It is important to note that protection was provided even though a very large dose of cells containing viruses was used for the challenge rather than a preparation of the virus particles alone which would have been a more usual procedure. Thus, protection of the cat hosts was demonstrated even under particularly stringent conditions.

VI. Antigens Related to fes- and fps-Oncoproteins in Human Neoplasia

A. Diagnosis

As a demonstration of the relation between viral oncoproteins and oncoproteins produced in human tumors, cell lysates from human cell lines were screened with rabbit antisera raised to the polypeptide II-KLH conjugate as described above. A 40 Kd oncoprotein was immunoprecipitated during those screenings from cell lysates of human lung carcinoma, human malignant melanoma, human epidermal carcinoma, human rhabdomyosarcoma and human fibrosarcoma. The 40 Kd oncoprotein was not immunoprecipitated from lysates of fresh human peripheral blood monocytes, fresh human peripheral blood lymphocytes or an established, cultured line of human lymphocytes.

As further evidence of the cross-reactivity of antibodies to the synthetic polypeptides of the present invention to oncoproteins produced in neoplastic conditions, antisera raised to the polypeptide II-KLH conjugate were individually contacted with sera from 48 human patients with various neoplastic diseases. The neoplasias of those patients included various carcinomas, multiple myeloma, sarcomas of different tissues, lymphosarcomas, chronic leucocytic and acute myelogenous leukemias and melanomas.

Competition ELISA assays (see Figure 6) with the above sera revealed the presence of a cross-reacting antigen(s) in about 70 percent of the human sera examined. The cross-reacting antigen(s) was not identified. Sera from three normal individuals; i.e., individuals having no diagnosed neoplastic disease, did not show the presence of a cross-reacting antigen.

The above results illustrate several points. First, they demonstrate the fact that antibodies to the synthetic polypeptides of the present invention recognize an oncoprotein-related product other than that oncoprotein encoded by the viral oncogene whose antigenic domain was the basis for the synthetic polypeptide.

Second, the antibodies raised to the synthetic polypeptide are relatively specific in not immunoprecipitating proteins present in normal cells or sera. This is in contrast to the work reported by Wong and Goldberg, supra, and Papkoff et al., supra.

The above results also take the work of Eva et al., supra, to a very useful, practical and unexpected further step. Thus, a body component such as serum, urine or a lysed tissue sample can now be readily assayed and used to diagnose the presence of neoplastic disease by admixing that body component with antidodies raised to the synthetic polypeptides or their conjugates of this invention and analyzing the admixture for the presence of an immunological reaction between the body component and the antibodies so raised. Several of such immunological reactions are discussed hereinafter.

The above diagnosis of neoplastic disease is a more practical approach than that of Eva et al. for several reasons. First, the relatively inconvenient isolation of poly(A)-containing RNAs as disclosed by Eva et al. is not needed. Second, the preparation of antisera, and synthetic polypeptides and their conjugates used in this invention are standard, known procedures. Third, storage and usage of the present antibody preparations are also standard, known techniques. Fourth, complicated technologies involving specialized reagents for nucleic acid hybridization are not necessary. Thus, such diagnosis can be a convenient laboratory tool.

The above diagnostic method was also unexpected from the report of Eva et al. Those authors only identified poly(A)-containing RNAs, not proteins. Thus, although the neoplastic cells transcribed oncogene-related DNAs into poly(A)-containing RNAs, it was not known or predicted whether those RNAs were themselves translated into oncoproteins in those cell lines studied by Eva et al. The execution of a gene function requires, in almost all cases, the

0119702

expression of the protein product encoded in the gene sequence. As such, the detection of oncogene protein products in cells or tissues provides a more direct diagnostic marker for the functioning of an oncogene. The present diagnostic technique avoids the unknown remaining from the report of Eva et al. and provides a diagnostic tool for detecting the presence of an oncogene transcript that is known to be important in neoplastic diseases, the oncoprotein.

B. Neoplasia and Additional Synthetic Polypeptides

While the above results were obtained using antibodies to the fes-related polypeptide II-KLH conjugate, detection of a broader range of neoplastic diseases may be obtained by use of specific antibodies raised to additional antigenic polypeptides or their conjugates. Exemplary of such antigenic polypeptides to which the antibodies may be raised include those of Table 3, hereinbelow, that are synthesized using the previously described method to correspond to antigenic domains on the oncoproteins encoded by the fes, fps, ras, src, yes, myb, sis and mos viral oncogenes. The polypeptides are listed in Table 3 by oncogene family, with various polypeptides being paired with a corresponding polypeptide from a homologous antigenic domain of a related oncoprotein, where appropriate.

## Table 3

### Synthetic Antigenic Polypeptides

#### Group A - fes/fps Family

| | | |
|---|---|---|
| 1. | fes | S-P-Y-P-N-L-S-N-Q-Q-T-R-C |
| | fps | V-P-Y-A-N-L-S-N-Q-Q-T-R-C |
| 2. | fes | C-Y-Q-E-L-Q-S-I-R-K-R-H-R |
| | fps | C-H-Q-D-L-I-A-I-R-K-R-H-R |
| 3. | fes | C-P-V-K-W-T-A-P-E-A-L-N-Y-G-R |
| | fps | C-P-V-K-W-T-A-P-E-A-L-N-Y-G-W |
| 4. | fes | C-R-E-T-L-P-P-D-I-K-A-K-F-L |
| | fps | C-R-E-T-L-P-P-E-L-K-A-K-F-L |
| 5. | fes | C-V-P-K-D-K-W-V-L-N-G-E-D-L |
| | fps | C-V-L-K-D-K-W-V-L-N-H-E-D-V |
| 6. | fes | C-F-L-R-T-E-G-A-R-L-R-M-K-T |
| | fps | C-F-L-R-S-K-G-P-R-L-K-M-K-K |
| 7. | fes | S-D-V-W-S-F-G-I-L-L-W-E-A-F-S-L-G-A-V-P- |
| | fps | S-D-V-W-S-F-G-I-L-L-W-E-T-F-F-L-G-A-S-P- |
| | | Y-P-N-L-S-N-Q-Q-T-R-C |
| | | Y-A-N-L-S-N-Q-Q-T-R-C |
| 8. | fes | C-S-Q-A-E-A-S-K-D-K-D-R-D-K |
| | fps | C-K-Y-Q-E-A-S-K-D-K-E-R-E-K |
| 9. | fes | Q-R-V-K-S-D-R-E-Y-A-G-C |
| | fps | Q-R-A-K-S-D-R-E-Y-A-G-C |

#### Group B - ras Family

| | | |
|---|---|---|
| 10. | ras | Y-R-E-Q-I-K-R-V-K-D-S-D-D-V-P-M-V-L-V-G-N-K-C |
| 11. | ras | R-E-I-R-Q-H-K-L-R-K-L-N-P-P-D-E-S-G-P-G-C |
| 12. | ras | G-Q-E-E-Y-S-A-M-R-D-Q-W-M-R-T-G-E-G-F-L-C |
| 13. | ras | P-M-R-D-P-V-A-P-V-R-A-P-A-L-P-R-P-A-P-G-C |
| 14. | ras | Y-R-L-K-K-I-S-V-E-E-K-T-P-G-C |
| 15. | ras | Y-R-L-K-K-I-S-K-E-E-K-T-P-G-C |

#### Group C src/yes Family

| | | |
|---|---|---|
| 16. | src | C-W-R-R-D-P-E-E-R-P-T-F-E-Y-L |
| | yes | C-W-K-K-D-P-D-E-R-P-T-F-E-Y-I |
| 17. | src | G-E-V-L-D-R-V-E-R-G-Y-R-M-P-C |
| | yes | R-E-V-L-E-Q-V-E-R-G-Y-R-M-P-C |

18. src  V-E-R-M-N-Y-V-H-R-D-L-R-A-A-C̲
    yes  I-E-R-M-N-Y-I-H-R-D-L-R-A-A-C̲
19. src  E-A-Q-V-M-K-K-L-R-H-E-K-L-V-C̲
    yes  E-A-Q-I-M-K-K-L-R-H-D-K-L-V-C̲
20. src  K-G-L-N-Y-K-H-Y-K-I-R-K-L-D-S-C̲
    yes  R-G-D-N-V-K-H-Y-K-I-R-K-L-D-N-C̲
21. src  C̲-G-K-I-T-R-R-E-S-E-R-L-L
    yes  C̲-G-K-M-G-R-K-D-A-E-R-L-L

Group D myb

22. myb  C̲-F-H-K-D-Q-T-F-T-E-Y-R-K-M-H-G
23. myb  R-H-Y-T-D-E-D-P-E-K-E-K-R-I-K-E-L-E-L-C̲
24. myb  R-R-K-V-E-Q-E-G-Y-P-Q-E-S-S-K-A-C̲

Group E sis

25. sis  I-R-T-V-R-V-R-R-P-P-K-G-K-H-R-K-C
26. sis  C-K-H-T-H-D-K-T-A-L-K-E-T-L
27. sis  C̲-G-D-P-I-P-E-E-L-Y-K
28. sis  V-R-K-K-P-I-F-K-K-A-T-V-T-L-E-D-H-L-A-C
29. sis  L-Q-G-D-S-G-K-E-D-G-A-E-L-D-L-C̲

Group F mos

30. mos  R-Y-L-P-R-E-L-S-P-S-V-D-S-R-S-C
31. mos  P-R-R-L-A-W-F-S-I-D-W-E-Q-V-C
32. mos  C-T-E-D-L-R-A-S-Q-R-S-F-W-A-E-L
33. mos  C-S-Q-K-L-Q-D-L-R-G-R-Q-A-S-P-P-H
34. mos  T-H-Q-A-P-E-I-L-K-G-E-I-A-T-P-K-A-C̲

The above polypeptides are depicted from left to right in the direction from amino-terminus to carboxy-terminus. A virgule (/) separating two gene names indicates a relatedness or homology between determinant domains of the oncoproteins encoded by those genes, as well as similarity between the types of neoplasia induced in their natural hosts by infection with the appropriate viruses. Underlined Cys residues (C̲) found at either the amino- or the carboxy-termini of the above polypeptides are not present in the antigenic domains of the naturally occurring oncoproteins and are added during synthesis to the above polypeptides to facilitate bonding of the polypeptide to a carrier molecule.

The above synthetic polypeptides corresponding to the fes/fps and src/yes families of oncoproteins can also be written in the alternative, parenthesized, format as single sequences. Such sequences of those two groups of polypeptides are listed below in Table 4, with the added terminal cysteine residues (C) useful for bonding to the carrier removed for convenience. The synthetic polypeptides of Table 4 are written in the same order from amino-terminus to carboxy-terminus and bear corresponding numbers to those of Table 3.

### Table 4
### Synthetic Antigenic Polypeptides
### Written in Alternative Form

Group A-fes/fps Family

1. S(V)-P-Y-P(A)-N-L-S-N-Q-Q-T-R
2. Y(H)-Q-E(D)-L-Q(I)-S(A)-I-R-K-R-H-R
3. P-V-K-W-T-A-P-E-A-L-N-Y-G-R(W)
4. C-R-E-T-L-P-P-D(E)-I(L)-K-A-K-E(F)-L
5. V-P(L)-K-D-K-W-V-L-N-H-E-D-L(V)
6. F-L-R-T(S)-E(K)-G-A(P)-R-L-R(K)-M-K-T(K)
7. S-D-V-W-S-F-G-I-L-L-W-E-T(A)-F-F-L-G-A-S(V)-
   P-Y-P(A)-N-L-S-N-Q-Q-T-R
8. S(K)-Q(Y)-A(Q)-E-A-S-K-D-K-D(E)-R-D(E)-K
9. Q-R-V(A)-K-S-D-R-E-Y-A-G

Group C-src/yes Family

16. C-W-R(K)-R(K)-D-P-E(D)-E-R-P-T-F-E-Y-L(I)
17. G(R)-E-V-L-D(E)-R(Q)-V-E-R-G-Y-R-M-P-C
18. V(I)-E-R-M-N-Y-V(I)-H-R-D-L-R-A-A
19. E-A-Q-V(I)-M-K-K-L-R-H-E(D)-K-L-V
20. K(R)-G-L(D)-N-V-K-H-Y-K-I-R-K-L-D-S(N)
21. G-K-I(M)-T(G)-R-R(K)-E(D)-S(A)-E-R-L-L

C.  Improved Range of Neoplasia
Diagnosis, Prevention and Treatment

The above range of cross-reactivity demonstrated for antibodies raised to the polypeptide II-KLH conjugate can be widened for diagnostic, prevention, treatment and other purposes by admixture of antibodies or other, relatively smaller idiotype-containing polyamides raised to a plurality of different synthetic polypeptides. Intact antibodies are the preferred idiotype-containing polyamides and are used illustratively hereinafter in this section.

Here, first antibodies are raised to a first polypeptide or its conjugate whose amino acid residue sequence corresponds to a detminant domain of a first oncoprotein. At least a second group of antibodies is then raised to a second synthetic polypeptide whose amino acid residue sequence corresponds substantially to a determinant domain of: (i) a second oncoprotein that is unrelated to the first oncoprotein, or (ii) a domain of the first oncoprotein that is different from the domain of the first polypeptide, or (iii) a second oncorportein that is related to the first oncoprotein, but wherein the determinant domain of the second oncoprotein corresponding to the second synthetic polypeptide is other than homologous to the determinant domain of the first oncoprotein.

For example, a mixture of antibodies raised to synthetic polypeptides corresponding to antigenic domains of oncoproteins encoded by the fps and src genes provides a wider range of cross-reactivities than do the antibodies raised to fps alone. Exemplary different antigenic domains of single oncoproteins, e.g. ras-related polypeptides 10-15, are shown in Table 3, hereinabove, as are exemplary paired antigenic domains of related oncoproteins, e.g. src - and yes-related polypeptides 16-22 of Table 3.

The range of cross-reactivity can be broadened still further by use of mixtures of antibodies raised to synthetic polypeptides corresponding to oncoproteins encoded by viral genes which cause different types of neoplasia in their hosts. One such mixture of a plurality of antibody types is a mixture of antibodies raised to synthetic polypeptide II (fps-related) and those raised to one of the synthetic polypeptides in Table 3 raised to the myb-encoded oncoprotein. Thus, specific antibodies raised to a sarcoma-related viral oncoprotein encoded by the fps gene are present along with specific antibodies raised to a leukemia-related viral oncoprotein.

D. Diagnostic Systems

The above discussed antibody-oncoprotein reactivities and cross-reactivities provide the basis for a diagnostic system for assaying for the presence of neoplasia in an animal by detecting an increased amount of an oncoprotein encoded by an oncogene normally present in cells compared to the amount of that oncoprotein normally expressed.

One diagnostic system contains at least two reagents in biologically active form present in separate containers. The first reagent is a synthetic polypeptide of this invention whose amino acid residue sequence corresponds to a determinant domain of a first oncoprotein produced by cells transformed by an oncogenic virus. The second reagent includes idiotype-containing polyamides of this invention raised to the synthetic polypeptide. An indicating group is also present.

In some diagnostic systems of this invention in which indicating groups are bonded or otherwise bound to idiotype-containing polyamides, molecules relatively smaller than intact antibodies are sufficient for use. However, it is preferable to utilize intact antibody molecules in other diagnostic systems. Exemplarly of such latter systems are (1) those in which indicating groups are bound to second antibodies raised in a second animal to the first antibodies such as where goat anti-rabbit antibodies contain the indicating group, and (2) those wherein protein A alone or with an additional indicating group is utilized. The term "antibody" in its various grammatical forms will be used in sections E and F hereinbelow as illustrative of an idiotype-containing polyamide.

Admixture of predetermined amounts of the first and second reagents provides a predetermined amount of immunoreaction. Admixture of predetermined amounts of the first and second reagents in the presence of a predetermined amount of body component to be assayed provides another amount of immunoreaction. The amount of the latter immunoreaction is different when the body component contains a second oncoprotein having an antigenic domain homologous to that of the first oncoprotein that is present in an increased amount, compared to a known immunoreaction amount for the normal level of that oncoprotein in the body component, or to that amount produced by admixture of the first and second reagents alone.

### E.    Immunoreactions

The immunoreactions utilizing specific antibodies raised to the above polypeptides or their conjugates are widely varied. Several useful immunoreactions for the above diagnostic system are discussed hereinafter.

1. ELISA

Among the useful immunoreactions, one of the best known and easiest to carry out is the well known technique of competitive enzyme-linked immunosorbent assay or ELISA. An exemplary ELISA has been described previously in Sections III and IV.

Briefly, however, microtiter test wells were coated with a predetermined amount of a synthetic polypeptide. Antibodies such as well characterized rabbit immunoglobulins directed against the polypeptide serve as the specific antiserum. An enzyme such as horseradish peroxidase, alkaline phosphatase or the like was bonded to the antibody molecules following usual procedures to serve as the indicating group.

A dilution of the serum which provided about 30-50 percent of the saturation ELISA reading (Figure 6A) was then prepared. Ten microliters of the diluted antiserum was contacted and incubated with 90 microliters of the sample of body component to be assayed, e.g., patient serum, tissue extract, lyophilized and desalted urine or the like. A blank control was provided by 10 microliters of the diluted serum contacted and incubated with 90 microliters of a 3% solution of bovine serum albumin.

The presence of the oncoprotein to which the original polypeptide is related was reflected by a change in the ELISA curve of the standard antiserum after it had been incubated with a candidate test sample. An exemplary assay is shown using the synthetic polypeptide II, antiserum raised to its KLH conjugate and extracts from FeSV-infected cells in Figure 6B. The amount of oncoprotein in the sample can be calculated from the amount of inhibition of the polypeptide-antibody reaction observed, and that amount of oncoprotein present in the body component over the usually present amount can be calculated thereafter.

The above assay is relatively rapid to perform, taking less than 8 hours, and does not require the use of radioisotopes. It is also relatively sensitive, detecting as little as about 10 to about 50 nanograms of oncoprotein per milliliter of serum, urine, tissue extract or other body component. In addition, this assay is not significantly affected by the presence of proteolytic, degradative enzymes present in serum, tissue extract or urine samples since even after a partial degradation of the native oncoprotien, sufficiently large antigenic domains remain to be detected.

The above assay was utilized in the before described results in which antibodies to the synthetic dodecapeptide II revealed the presence of a cross-reacting antigen in 70 percent of sera from patients known to have various neoplastic diseases. Again, the breadth of cross-reactivities obtained by use of mixtures of antibodies raised to a plurality of different antigenic domains of oncoproteins can be availed of to provide a wider qualitative analysis for the presence of different oncoproteins.

2. Radioimmunoassay

Radioimmunoassays can also be used qualitatively and quantitatively to detect the presence of increased levels of oncoproteins in body compenents. Here, a selected, synthetic oncoprotein-related polypeptide such as one of those listed in Table 3 is provided. Antibodies are raised to that polypeptide, or its conjugate, and are collected. The synthetic polypeptide is radiolabeled, as by the method of Hunter et al., Nature, 194, 495-496 (1962).

The amounts of radiolabeled polypeptide and antibody are adjusted so that the antibodies in the antiserum precipitate about 30-50% of the radioactivity available from the labeled synthetic polypeptide. Approximately 10 nanograms of synthetic polypeptide labeled to contain about 10,000 disintegrations per minute (dpm) of radioactivity and having specific activity of about 1,000,000 dpm per microgram are typically used. The amount of antibodies is typically adjusted by serial dilutions from standard antiserum in buffered saline. A convenient amount of precipitated radioactivity is about 3000 to about 5000 dpm.

A predetermined amount of antibody is admixed and incubated for a predetermined time with a predetermined amount of body component to be assayed. The radiolabeled synthetic polypeptide to which the antibodies were raised is then admixed with the antibody-body component admixture. Reduction in the amount of radioactive polypeptide precipitated from the predetermined amount (e.g., 30-50 percent of 10,000 dpm) provides a measure of the amount of oncoprotein having a homologus antigenic domain to that polypeptide used to raise the antibodies that was present in the body component. The use of mixed antibody types, as discussed above, can provide a wider breadth for qualitative assays for the presence of oncoproteins.

### 3. Blot Analysis

Body components such as urine or serum samples, or tissue preparation can also be subjected to polyacrylamide gel electrophoresis under denaturing, reducing conditions. The separated proteins are then transferred to a nitrocellulose membrane by the application of an electric field. [Towbin et al., Proc. Natl. Acad. Sci. U.S.A., 76:4350-4354 (1979)].

Antibodies raised to synthetic polypeptides such as those of Table 3 are then contacted and incubated with the nitrocellulose sheets. The presence of an oncoprotein in the body component is detected by contacting the body component-antibody-incubated nitrocellulose sheet with a preparation of radiolabeled protein A, commercially available in purified form derived from Staphylococcus Aureus. Protein A binds to the body component-antibody complex, as is well known. Chromophore-bonded protein A can be used instead of the radiolabeled protein to provide a color-based assay rather than an assay based on radioactivity.

The above technique provides a qualitative assay for the presence or absence of an oncoprotein related to the synthetic polypeptide. It also provides information as to the size of the oncoprotein molecule from its relative position after electrophoresis. As is the case for the ELISA and radioimmunoassays, the use of antibodies raised to a plurality of synthetic polypeptides provides a wider range of cross-reactivities and therefore a wider spectrum of possible positive, qualitative results.

F. Neoplastic Cell Location

Oncoproteins such as pp60$^{src}$ are found on cell surfaces. Use of this fact can be made by raising antibodies to the synthetic polypeptides of this invention corresponding to antigenic domains of such oncoproteins or to homologous domains of other oncoproteins, or to conjugates made with them, and labeling those antibodies or their Fab or F(ab')$_2$ fragment portions (idiotype-containing polyamides) appropriately. The labeled antibodies can then be utilized to locate cells containing oncoproteins having antigenic domains that are cross-reactive with the antibodies so raised.

### 1. In vitro Analysis

In one in vitro analysis, antisera were raised in rabbits to a synthetic polypeptide conjugate of this invention, the polypeptide II-KLH preparation discussed hereinbefore. As an exemplary system, cultured cells, a body component, made cancerous by infection with FeSV and normal cells were grown on separate microscope cover slides as cell spreads. These cells were then contacted and incubated with the above rabbit anti-synthetic polypeptide antiserum.

Previously prepared goat anti-rabbit antiserum bonded to fluoresein by known techniques was obtained from comercial sources and incubated with each cell spread-rabbit antiserum incubate. Irradiation with light of appropriate wavelength using a color filter in a fluorescence microscope visualized fluorescent areas on and/or in infected cells where the oncoprotein formed a complex with the rabbit antibodies, and the complex so formed bound the fluorescin-labeled goat antirabbit antibodies, thereby locating the cellular cross-reactive oncoprotein. Thus, the cellular location of the oncoprotein was obtained by the technique termed "fluorescence microscopy". Fluorescence microscopy can also be used to identify and locate cross-reactive oncoproteins encoded by related viral genes or by cellular oncogenes in tumor specimens.

2.  In vivo Analysis

Several in vivo methods are also available for locating the presence of cells containing an increased level of oncoproteins. In one such method, antibodies are raised to one or a plurality of synthetic polypeptides. The antibodies so raised are labeled with an indicating group.

An exemplary indicating group is a radioactive element that produces gamma ray emissions. Elements which themselves emit gamma rays such as $^{125}I$ represent one class of gamma ray emission-producing radioactive element indicating groups. Another class of useful indicating groups are those elements such as $^{11}C$, $^{18}F$, $^{15}O$ and $^{13}N$ which themselves emit positrons. The positrons so emitted produce gamma rays upon encounters with electrons present in the animal's body.

The radiolabeled antibodies are then injected into the blood stream of an animal having a neoplastic disease, especially a tumor. After a suitable, predetermined time, the labeled antibodies form complexes with the related oncoproteins on the tumor surface. The animal is scanned with a gamma ray emission counting machine such as the axial tomagraphic scanner commercially available under the designation CT (80-800 CT/T) from General Electric Company, Milwaukee, WI, or with a positron emission transaxial tomography scanner such as that designated Pett VI located at Brookhaven National Laboratory. Such scanning can provide information as to the location, size and shape of the tumor because of the specificity of the radiolabeled antibodies utilized.

In another embodiment, antibodies to the synthetic polypeptides or their conjugates are again raised. Here, the antibodies are labeled with an indicating group containing an element that is active in nuclear magnetic resonance (NMR) spectroscopy; i.e., an NMR-active element. Many such elements are commercially available in addition to the more usual $^{13}C$, $^{15}N$, and the like.

It is particularly preferred to utilize an indicating group containing the NMR-active $^{19}F$ atom, or a plurality of such atoms inasmuch as (i) substantially all of naturally abundant fluorine atoms are the $^{19}F$ isotope and thus substantially all fluorine-containing compounds are NMR-active; (ii) many chemically active polyfluorinated compounds such as trifluoroacetic anhydride are commercially available at relatively low cost, and (iii) many fluorinated compounds have been found medically acceptable for use in humans such as the perfluorinated polyethers utilized to carry oxygen as hemoglobin replacements.

Another particular advantage of the use of fluorine-containing NMR-active indicating groups is that the body contains very little fluorine under normal conditions. Consequently, by using an NMR-active element that is otherwise substantially absent from the animal, background signals due to bodily fluorine atoms are substantially absent. Thus, the principal signals observed are due to the labeled anti-polypeptide-neoplastic cell complexes.

In this embodiment, the antibodies are preferably labeled with a fluorine-contianing material such as trifluoroacetic anhydride or hexafluoroethanol to form a fluorinated amide or ester derivative, respectively. Thereafter, the fluorinated antibodies are injected into the bloodstream of the tumor-containing animal. After a predetermined amount of incubation time for the labeled antibodies to complex with the related oncoprotein on the tumor cell surface, a so-called "whole-body" NMR determination is carried out using an apparatus such as one of those described by Pykett, Scientific American, 246, 78-88 (1982) to locate the tumor.

Thus, the above methods of locating a neoplastic tumor in vivo in an animal include the steps of:

(a)   providing a composition comprising idiotype-containing polyamides such as antibodies raised to the synthetic polypeptides of this invention, or their conjugates bound to a carrier, wherein the idiotype-containing polyamides are bonded individually to indicating groups.  The useful indicating groups include gamma ray emission-producing elements, NMR-active elements and the like.

(b)   The composition so provided is injected into the bloodstream of a neoplastic tumor-bearing animal.

(c)   The animal so injected is incubated for a predetermined period of time sufficient for the indicating group-bonded anti-polypeptide to form a complex on the surface of the tumor.

(d)   The animal is then scanned with a means for detecting the location of the complexed indicating group.  Typical detecting means include usually used gamma ray emission detectors, those machines used in positron emission tomography and so-called "whole body" NMR spectrometers which may in practice only scan a portion of the body at any time.

VII.   Treatment of Neoplastic Diseases

The discussion in Section V, above, related to the use of idiotype-containing polyamides such as antibodies raised to a polypeptide II-KLH conjugate to immunize and protect a host (cats) against subsequent infection by FeSV-containing cells that were injected into the animals. The present discussion relates to use of idiotype-containing polyamides, and particularly to antibodies raised to the synthetic polypeptides of this invention or their conjugates to treat neoplasia that is known to be present in cells. Here again the selective cross-reactivity of the idiotype-containing polyamides to oncoproteins different from but related to the oncoprotein domains to which they were raised is utilized. The specificity of the antibodies of this invention to domains vicinal to the active site of the oncoprotein can also be utilized. Antibodies are again used as exemplary of idiotype-containing polyamides in Sections A and B hereinbelow.

A. Antibody-Cell Reactions

Antibodies raised to a polypeptide of this invention or its conjugate are provided, as are neoplastic cells, either in vivo or in vitro. The antibodies and neoplastic cells are admixed to contact the cells with the antibodies. Such contact causes formation of an immune complex between the antibodies and the related cellular oncoprotein having a homologous antigenic domain, and can block an active site of the cellular oncoprotein.

Blockage of a cellular oncoprotein's active site prevents or retards its function in oncogenesis and thereby provides an abatement of the oncogenic effect on the cells. Such abatement of function provides a stasis in the oncogenic condition for cells in vitro and thereby inhibits the growth of neoplastic cells.

The formation of immune complexes on cell surfaces _in vivo_ or _in vitro_ where the members of the complement pathway system are available leads to lysis of the neoplastic cells containing immune complexes on their surfaces. This lysis occurs in the same way that the members of the complement pathway system lyse invading cells under usual immune system reaction conditions and is well known in the art.

B.  Cytotoxic-Agent Linked Antibodies

In yet another embodiment of this invention, a cytotoxic agent is linked to the antibodies of this invention. The cytotoxic agent acts to kill the neoplastic cells in addition to any action provided by the antibodies or their idiotype-containing fragments. Thus, the specificity of the antibodies for locating and binding to neoplastic cells containing cross-reactive oncoproteins is again utilized.

The cytotoxic agent utilized can be a drug such as adriamycin, the G-418 variant of neomycin or the like. It is particularly preferred that the cytotoxic agent act at least in part by contact with neoplastic cell membranes.

Adriamycin is exemplary of a particularly preferred cytotoxic agent. The adriamycin is bonded to an antibody through its sugar ring-amino group via a dialdehyde such as glutaraldehyde, by a water-soluble carbodiimide or by other well known linking means. Tritton et al., _Science_, 217, 248-249 (1982) reported linking of adriamycin to agarose beads.

Those authors bonded the drug to the agarose beads as a means of contacting neoplastic cell membranes while avoiding general dissemination of the drug throughout the body tissues since adriamycin is known to have toxic effects upon the hearts of cancer patients. The cancer cells so contacted by Tritton et al. died.

It was suggested in an article appearing on page 69 of Science News, July 31, 1982 that a means other than the agarose beads would be needed for in vivo use on human patients. However, that suggestion was limited to one specific material, plasma membrane proteins, and did not include the use of the specific antibodies raised to the present synthetic polypeptides or their conjugates, inasmuch as antibodies such as those disclosed herein have not heretofore been available.

VIII. Monoclonal Idiotype-Containing Polyamides

The above discussion has dealt with idiotype-containing polyamides such as antibodies that have specific activity against a determinant domain in one oncoprotein as well as activities against homologous domains in other oncoproteins. However, where a synthetic polypeptide is conjugated to a carrier, antibodies directed against the carrier can also be raised. In addition, the individual antibody molecules that are raised to the synthetic polypeptide portions of the conjugate can have varying immunoreactivities with the synthetic polypeptide and/or oncoprotein.

The presence of anti-carrier antibodies and antibodies having varying reactivities with the synthetic polypeptide and/or oncoprotein in antisera that also contain the strongly immunoreacting specific antibodies to synthetic polypeptides of the invention tends to dilute the activity of the latter antibodies and their relatively smaller idiotype-containing polyamide portions, based upon the total number of idiotype-containing molecules present. Monoclonal idiotype-containing polyamides were therefore prepared to raise the per molecule immunoreactivity of the desired idiotype-containing polyamides. Intact antibodies were again used as exemplary of idiotype-containing polyamides.

Mice were immunized with the polypeptide II-KLH conjugate discussed hereinbefore, using 50 micrograms of conjugate in complete Freund's adjuvant per animal. One week after the first immunization each animal was given a booster immunization containing another 50 micrograms of the conjugate in incomplete Freund's adjuvant.

Antibody titers were followed with test bleeds using the ELISA technique discussed hereinbefore until the titer reached a value of at least about 1:1000 to about 1:5000. Once the titers reached the desired 1:1000-5000 levels, the animals were allowed to rest for about 3 to 4 weeks.

Thereafter, each animal was again boosted with a third immunization. The third immunizing solution contained 500 micrograms of conjugate in either water or water containing 10 percent alum.

The animals were sacrificed 72-120 hours after the third immunization and their spleens were removed. The spleen cells so obtained were fused, and monoclonal antibodies were prepared using well characterized procedures. See U.S. Patent No. 4,172,124.

The monoclonal antibodies so produced were screened by ELISA for their immunoreactivities with polypeptide II using supernatant from the cultured cells as the antibody source. Those monoclonal antibodies exhibiting strong immunoreactivities were then tested for their ability to immunoprecipitate the _fes_ oncoprotein. The cell lines providing monoclonal antibodies that immunoreact strongly with the synthetic polypeptide and immunoprecipitate its corresponding oncoprotein are then propagated and their monoclonal antibodies are collected for use.

The foregoing is intended as illustrative of the present invention but not limiting. Numerous variations and modifications may be effected without departing from the true spirit and scope of the novel concepts of the invention. It is to be understood that no limitation with respect to the specific polypeptides, antibodies, their compositions and uses illustrated herein is intended or should be inferred. The invention is defined by the claims which follow.

CLAIMS:

1. A synthetic polypeptide having an amino acid residue sequence corresponding substantially to an amino acid residue sequence of a determinant domain of an oncoprotein encoded by an oncogenic virus, said determinant domain being vicinal to but exclusive of an active site of said oncoprotein, and said synthetic polypeptide having the capacity, alone or as a conjugate of said polypeptide bound to a carrier, of inducing the production of antibodies when injected into a host, said antibodies inhibiting a reaction at said active site of said oncoprotein.

2. The synthetic polypeptide according to claim 1 containing about 10 to about 40 amino acid residues.

3. The synthetic polypeptide according to claim 1 wherein said sequence contains about 10 to about 25 amino acid residues.

4. The synthetic polypeptide according to claim 1 having an amino acid sequence containing about 10 to about 25 amino acid residues and corresponding substantially to an amino acid residue sequence of a first determinant domain of a first oncoprotein produced by cells transformed by an oncogenic virus, said first determinant domain being vicinal to but exclusive of a phosphotransfer catalysis site of said oncoprotein, and said synthetic polypeptide having the capacity, alone or as a conjugate of said polypeptide bound to a carrier, of inducing the production of antibodies when injected into a host animal, wherein said antibodies:

(a) immunoprecipitate said oncoprotein;

(b) inhibit a phosphotransfer reaction catalyzed by said oncoprotein at said phosphotransfer site; and

(c) react competitively with an admixture containing said polypeptide or said conjugate and a second oncoprotein present in a body component of a tumor-bearing animal, said second oncoprotein having a determinant domain homologous to said first domain, and the tumor of said animal being other than that induced by said oncogenic virus.

5. The synthetic polypeptide of any preceding claim bonded to a carrier molecule.

6. An antibody raised to a synthetic antigen, said synthetic antigen comprising a synthetic polypeptide of any preceding claim, or a conjugate of said polypeptide bound to a carrier.

7. The antibody according to claim 6 that:

(a) inhibits a phosphotransfer reaction at an active site of a first oncoprotein,

(b) is capable of precipitating said first oncoprotein, and

(c) immunoreacts with a second oncoprotein having a determinant domain homologous to that of said first oncoprotein.

8. A diagnostic reagent system for assaying for the presence of an increased amount of a first oncoprotein encoded by an oncogene normally present in cells as compared to the amount of said first oncoprotein normally present, comprising in separate containers:

(a) a first reagent that contains in biologically active form a synthetic polypeptide of any of claims 1 to 4; and

(b) a second reagent in biologically active form including idiotype-containing polyamides raised to said synthetic polypeptide or its conjugate, along with indicating groups;

said first and second reagents when admixed in predetermined amounts in the presence of a predetermined amount of a body component to be assayed providing an amount of immunoreaction, the amount of said immunoreaction being different from a known immunoreaction amount when an increased amount of said first oncoprotein is present in said body component.

9. A diagnostic reagent comprising idiotype-containing polyamides that are individually bonded to indicating groups, wherein said idiotype-containing polyamides

(a) are raised to a synthetic polypeptide of any of claims 1 to 4, or to a conjugate of said synthetic polypeptide bound to a carrier; and

(b) immunoreact with a second oncoprotein having an antigenic domain homologous to said determinant domain of said first oncoprotein.

10. A method of inhibiting the growth of neoplastic cells comprising the steps of:

(a) providing neoplastic cells having oncoproteins on their surfaces;

(b) contacting said cells with an effective amount of idiotype-containing polyamides raised to a synthetic polypeptide of any of claims 1 to 4, or to a conjugate of said polypeptide bound to a carrier, said synthetic polypeptide having an amino acid sequence that is homologous to a determinant domain of said oncoproteins produced by said neoplastic cells; and

maintaining said contact to form immune complexes between said neoplastic cell surface oncoproteins and said idiotype-containing polyamides.

11. The method according to claim 10 wherein said idiotype-containing polyamides include a cytotoxic drug bonded thereto.

12. A method for detecting the presence of neoplastic disease in an animal comprising the steps of

(a) providing a body component from said animal;

(b) admixing said body component with a composition including idiotype-containing polyamides raised to a synthetic polypeptide of any of claims 1 to 4, or to a conjugate of said polypeptide bound to a carrier; and

(c) determining the presence, in said admixture, of an immunological reaction between said body component and said idiotype-containing polyamides.

13. A composition comprising idiotype-containing polyamides that are individually bonded to indicating groups, wherein said idiotype-containing polyamides

(i) are raised to a synthetic polypeptide of any of claims 1 to 4, or to a conjugate of said synthetic polypeptide bound to a carrier;

(ii) inhibit a reaction at an active site of said oncoprotein; and

(iii) immunoreact with a second oncoprotein having an antigenic determinant domain homologous to said domain of said first oncoprotein, for use in a method of locating a neoplastic tumor in an animal in vivo, said method comprising the steps of:

(a) injecting said composition into the bloodstream of a neoplastic tumor-bearing animal;

(b) incubating said animal for a predetermined period of time sufficient for the indicating group-bonded polypeptide to form a complex on the surface of said tumor; and

(c) scanning said animal with a means for detecting the location of said complexed indicating groups.

14. A vaccine comprising an effective amount of a synthetic polypeptide of any of claims 1 to 4, or a conjugate of said polypeptide bound to a carrier, dispersed in a physiologically tolerable vehicle.

15. A vaccine according to claim 14, for use in a method of immunising an animal against neoplastic growth.

16. An idiotype-containing polyamide raised to a synthetic polypeptide of any of claims 1 to 4, or to a conjugate of said polypeptide bound to a carrier, for use in a method of inhibiting the growth of neoplastic cells according to claim 10 or claim 11.

# FIG. 1

## IMMUNOPRECIPITATIONS

0119702

2/6

# FIG. 2

## INHIBITION OF PHOSPHOTRANSFER

**A**

**B**

FIG. 3

INHIBITION OF PHOSPHOTRANSFER

A

B

3/6

0119702

## Fig.4.

### ANTI-SYNTHETIC POLYPEPTIDE ANTIBODY TITERS

ABSORBANCE AT 413 NM

1.2
1.0
0.8
0.6
0.4
0.2

1/12,800   1/6,400   1/3200   1/1600   1/800   1/400   1/200

DILUTIONS OF STANDARD SERA

## FIG. 5
IMMUNOPRECIPITATION OF 40 Kd ONCOPROTEIN FROM HUMAN TUMORS

_Fig.6._

ELISA FOR NATIVE ONCOPROTEIN